# FASCICULE DE BREVET EUROPEEN

(11) **EP 3 281 013 B1**
(45) Date de publication et mention de la délivrance du brevet: **06.02.2019**
(21) Numéro de dépôt: 16731225.5
(22) Date de dépôt: 25.05.2016
(51) Int. Cl.: G01N 33/543

(54) **MÉTHODE ET LABORATOIRE POUR LE DIAGNOSTIC RAPIDE ET DE PROXIMITÉ DE MALADIES AIGUËS**
VERFAHREN UND LABOR ZUR SCHNELLEN UND LOKALEN DIAGNOSE AKUTER ERKRANKUNGEN
METHOD AND LABORATORY FOR RAPIDLY AND LOCALLY DIAGNOSING ACUTE DISEASES

(30) Priorité: 28.05.2015 FR 1554790
(43) Date de publication de la demande: 14.02.2018
(73) Titulaire: Pocramé, 13400 Aubagne (FR)
(72) Inventeur: DRANCOURT, Michel, 13012 Marseille (FR); LEVY, Pierre-Yves, 13008 Marseille (FR); RAOULT, Didier, 13008 Marseille (FR); SAMAD, Marc Abdul, 13009 Marseille (FR)
(74) Mandataire: Domange, Maxime
(86) Numéro de dépôt international: PCT/FR2016/051229
(87) Numéro de publication internationale: WO 2016/189248

(56) Documents cités:
- GB-A- 2 439 780
- MEHDI BOURICHA ET AL: "Point-of-Care Syndrome-Based, Rapid Diagnosis of Infections on Commercial Ships", JOURNAL OF TRAVEL MEDICINE, vol. 21, no. 1, 3 janvier 2014 (2014-01-03), pages 12-16, XP055226198, CA ISSN: 1195-1982, DOI: 10.1111/jtm.12090 cité dans la demande
- ALIMUDDIN ZUMLA ET AL: "Rapid point of care diagnostic tests for viral and bacterial respiratory tract infections-needs, advances, and future prospects", LANCET INFECTIOUS DISEASES, vol. 14, no. 11, 1 novembre 2014 (2014-11-01), pages 1123-1135, XP055226324, US ISSN: 1473-3099, DOI: 10.1016/S1473-3099(14)70827-8
- Management Médical ET AL: "En partenariat avec UN LABORATOIRE DE MICROBIOLOGIE DELOCALISE : UNE SOLUTION D'AVENIR POUR LES EXAMENS EN URGENCE ? ANALYSE MEDICO-ECONOMIQUE", , 1 janvier 2012 (2012-01-01), XP055226329, Extrait de l'Internet: URL:http://www.mediterranee-infection.com/ arkotheque/client/ihumed/_depot_arko/artic les/264/these-levy_doc.pdf [extrait le 2015-11-05]

## Description

La présente invention concerne une méthode d'aide à l'appréciation du pronostic et prise de décision d'évacuation, isolement ou traitement immédiat d'un patient, notamment dans un site isolé, par réalisation d'une série de tests de diagnostic in vitro de maladies aiguës, notamment infectieuses, à l'aide d'un laboratoire portatif et/ou mobile compact de réalisation de dits tests.

On entend ici par « laboratoire portatif et/ou mobile », un meuble contenant ou apte à contenir les matériels et réactifs requis pour la réalisation de tests biologiques de diagnostic, qui peut être déplacé et/ou transporté par une personne seule.

Le diagnostic direct des maladies infectieuses et tropicales, provoquées par des microorganismes parasites, champignons microscopiques, bactéries et virus, repose sur la mise en évidence dans un prélèvement cliniquement collecté chez le patient, du microorganisme entier par examen microscopique et culture ou un fragment spécifique de son matériel génétique constitué par de l'ADN, ARN ou les deux ou encore un ou plusieurs antigènes spécifiques du microorganisme. [Fournier PE et al. Modern Clinical microbiology: new challenges and solutions. Nat. Rev. Microbiol., 2013; 11:574-585].

Dans le mode habituel de mise en oeuvre de ces techniques dans un laboratoire de microbiologie, le délai de réponse est supérieur à 12 heures, du fait du délai d'acheminement des prélèvements cliniques jusqu'au laboratoire de microbiologie, ajouté au délai de mise en oeuvre des techniques de laboratoire. Le délai de mise en oeuvre des techniques de laboratoire est compris entre 1 heure et 48 heures, pour 95% des tests. Actuellement, le délai moyen de réponse par les laboratoires de microbiologie a tendance à augmenter, du fait du regroupement des laboratoires de biologie médicale et de microbiologie dans de grandes plateformes qui entraîne un éloignement des sites où sont réalisés les prélèvements cliniques et un allongement du délai d'acheminement.

Les résultats des tests de laboratoire de microbiologie guident le médecin et le personnel de santé en charge du patient, dans leurs décisions. Hors, le délai de réponse influence les modalités et la réussite de la prise en charge médicale du patient.

En particulier, l'éloignement des laboratoires de biologie médicale de centres hospitaliers universitaires (CHU), sur un seul site géographique, a entrainé un éloignement des moyens de réalisation de diagnostics pour les patients et leurs médecins, pouvant être dommageables lorsqu'une décision de traitement, isolement et/ou évacuation doit être prise rapidement pour éviter le risque mortel pour le patient.

Plus particulièrement, le médecin et le personnel de santé doivent répondre à trois questions suivantes qui guident la conduite à tenir vis-à-vis du patient :
1) Le patient est-il contagieux pour son entourage ? Si oui, le patient doit être isolé par des moyens et une durée appropriés à la maladie infectieuse contagieuse.
2) Le patient présente-t-il une maladie rapidement mortelle en l'absence de prise en charge médicale spécialisée ? Si oui, le patient doit être hospitalisé dans le délai le plus court possible, dans une structure médicale appropriée à la maladie.
3) Le patient présente-t-il une maladie curable par un traitement approprié ? Si oui, le patient doit bénéficier de ce traitement, dans le délai le plus court possible.

Parallèlement, cette situation d'éloignement du laboratoire de biologie médicale, se retrouve dans de nombreuses situations, où les médecins et d'autres professionnels sont demandeurs de diagnostic microbiologique plus rapide, pour une prise en charge la plus efficace et la moins couteuse possible des personnes suspectes d'infection contagieuse et dangereuse ou autres maladies aiguës requérant un avis médical et/ou un traitement ou des actions en urgence.

L'éloignement géographique observé dans certaines régions des pays développés difficiles d'accès, notamment des iles ou des villages de montagne et des pays en voie de développement, mais aussi dans des lieux de les rassemblements de masse lors de différentes manifestations sportives, culturelles, politiques et religieuses et dans les aéroports (1 milliard de passagers en 2013) sont des situations dans lesquelles les médecins, les professionnels non-médicaux et le public, sont demandeurs de nouvelles capacités de diagnostics ou pré diagnostics rapides pour optimiser la prise en charge médicale des patients fébriles. Il en va de même, en milieu maritime à bord des navires de commerce, navires militaires des Marines Nationales ainsi que dans les plateformes d'exploitation sous-marine telle que les plateformes offshore pétrolières ou autres.

On connait un type de laboratoire délocalisé dit borne de point-de-soin (ou « POC » pour « point-of-care ». Dans Bouchira et al (Journal of travel medecine 2014, vol 21 pages 12-16) on a proposé installation de laboratoire délocalisé avec la mise en oeuvre de kit syndromique c'est-à-dire adaptant le menu de certains tests rapides spécifiques à réaliser disponibles dans le commerce pour les maladies aiguës, notamment contagieuses, les plus dangereuses et/ou les plus fréquentes, en fonction du tableau clinique présenté par le patient et des circonstances épidémiologies particulières sur le site.

Cette installation de laboratoire de type POC comprend une simple hotte ouverte en face avant équipée d'un système de ventilation et filtration de l'atmosphère intérieure de la hotte qui ne permet pas de réaliser les tests d'analyse microbiologique dans un espace confiné et décontaminable.

D'autre part dans Bouchira et al 2014, l'opérateur doit de lui-même déterminer et choisir les kits syndromiques dont les tests doivent être réalisés.

Le but de la présente invention était donc de développer un mode d'organisation amélioré du laboratoire de biologie, pour un diagnostic ou pré diagnostic de sécurité rapide de proximité des maladies aiguës, notamment maladies infectieuses et tropicales.

Pour ce faire, les inventeurs ont conçu et développé une nouvelle installation et une nouvelle méthode plus sécurisée et plus facile à mettre en oeuvre applicable dans des sites isolés, impliquant la mise en oeuvre d'un laboratoire compact miniaturisé portatif et/ou mobile, sous forme de borne ou cabine, délocalisé au contact des patients et de leurs médecins ou personnes en charge de la population dans des sites géographiques isolés.

Plus particulièrement, les inventeurs ont conçu et développé une méthodologie et des équipements impliquant la mise en oeuvre d'un programme d'ordinateur pour la mise en oeuvre de ce laboratoire mobile compact selon une approche syndromique.

Plus précisément, la présente invention fournit un dispositif de laboratoire utile pour la mise en oeuvre d'une méthode de diagnostic comprenant la réalisation d'une série de tests d'analyses biologiques in vitro pour le diagnostic de maladies aiguës requérant une intervention médicale urgente comprenant au moins une pluralité de maladies infectieuses aiguës comprenant :
- k types d'enveloppes contenant chacune m trousses de tests d'analyse biologique, les dites m trousses de tests d'analyses biologiques permettant la détection chacune de respectivement m pathologies de k catégories de syndromes de dites maladies aiguës comprenant au moins des catégories de syndromes d'infections par des microorganismes pathologiques, les dites catégories de syndromes de dites maladies étant associées chacune à un syndrome différent affectant un organe ou tissu corporel, k étant un nombre entier supérieur à 2, de préférence k étant au moins égal à 6 catégories, m étant un nombre entier de préférence de 1 à 5, m pouvant être différent pour chaque dite catégorie de syndrome de dite maladie,
- une enceinte équipée d'un plan de travail, au sein de laquelle enceinte on peut réaliser les dits tests d'analyses biologiques in vitro, et
- un mobilier de rangement comprenant au moins une armoire isolée thermiquement, ladite armoire contenant ou apte à contenir une pluralité de dites enveloppes contenant chacune au moins une dite trousse de test d'analyse biologique, le dit mobilier de rangement étant disposé sous de ladite enceinte,

Le dispositif étant caractérisé en ce qu'il se présente sous forme d'une cabine portative et/ou mobile, transportable manuellement par une personne seule, comprenant :
- une enceinte fermée en face avant par un capot transparent amovible ou pivotant, munie d'au moins une ouverture d'accès pour les deux bras d'un opérateur, au sein de laquelle enceinte un opérateur peut réaliser les dits tests d'analyse biologique in vitro sur ledit plan de travail en position debout, dans un espace confiné avec ledit capot et
- un ordinateur, de préférence sous forme de tablette, disposé au sein de la dite enceinte au-dessus dudit plan de travail, équipé d'un programme d'ordinateur apte à assister l'opérateur pour la réalisation des dits tests d'analyses biologiques ou d'un support d'enregistrement lisible par ledit ordinateur sur lequel est enregistré un dit programme d'ordinateur; le dit programme d'ordinateur étant configuré pour l'exécution d'une série d'étapes guidant le choix et la réalisation ainsi que l'interprétation des résultats desdits tests d'analyses biologiques et les actions à entreprendre et/ou conduite à respecter pour le patient et/ou le personnel de son entourage en fonction des résultats des tests, comprenant au moins la succession des dites étapes suivantes comprenant :
   - e1) l'enregistrement de la sélection de signes cliniques et/ou symptômes ressentis par le patient choisis parmi n signes ou symptômes enregistrés dans ledit programme d'ordinateur, pouvant être ressentis par des patients atteints d'au moins une des m pathologies de chacune des dites k catégories de syndromes de dites maladies, n'étant un nombre entier de préférence au moins égal à k, et
   - e2) la détermination et l'indication du ou des types de dites enveloppes contenant la (ou les) trousse(s) de test d'analyse biologique à réaliser en fonction de la dite sélection de l'étape e1), et
   - e3) la détermination et l'indication du ou des modes opératoires pour effectuer au moins un prélèvement d'échantillon biologique de fluide ou sécrétion corporelle dudit patient à analyser, à l'aide des matériels et réactifs contenus dans la (ou les) dites(s) enveloppe(s) déterminée(s) à l'étape e2) et/ou dans ledit mobilier de laboratoire, et
   - e4) la détermination et l'indication du ou des modes opératoires pour réaliser et interpréter les résultats desdits tests d'analyses biologiques contenus dans la (ou les) dites(s) enveloppe(s) déterminée(s) à l'étape e2), et
   - e5) l'enregistrement du résultat positif, négatif ou ininterprétable de chacun des dits tests d'analyses biologiques réalisés contenus dans la (ou les) dites(s) enveloppe(s) déterminée(s) à l'étape2), et
   - e6) la détermination et l'indication des actions à entreprendre et/ou conduite à respecter pour le patient et/ou les personnes de son entourage en fonction de la combinaison de résultats positifs ou négatifs ou ininterprétables de tous les tests réalisés enregistrés à l'étape e5).

Plus particulièrement, les dits enregistrements et dites instructions des étapes e1) à e6) comprenant lesdits enregistrements des étapes e1 et e5) et les dites déterminations et indications des étapes e2) à e4) et e6), peuvent être opérées ou fournies via une interface graphique de l'écran dudit ordinateur avec laquelle ledit opérateur peut interagir par contact tactile et/ou via un clavier, notamment pour sélectionner un choix ou opérer un enregistrement quand cela est requis.

Les instructions des étapes e1) à e6) peuvent être affichées sur une succession d'écran ou faire l'objet de fonctions séquentielles regroupées sur un même écran.

Le dispositif portatif selon la présente invention apporte une sécurité dans la réalisation des tests tant au niveau de la sécurité sanitaire de par la barrière physique de sécurité résultant des caractéristiques dudit capot de l'enceinte que par le guidage de l'opérateur par le programme d'ordinateur pour la réalisation de la méthodologie des tests.

La cabine selon la présente invention assure une protection de l'opérateur et de l'environnement sans que le matériel et le procédé ne doivent être mise en oeuvre dans une pièce isolée biologiquement comme c'est le cas dans les POC usuels.

De préférence, pour faciliter la réalisation sécurisée des dits tests y compris la gestion des déchets et matériels usagés, la dite cabine laboratoire comprend :
- une partie supérieure comprenant la dite enceinte avec un dit capot munie de deux premiers orifices circulaires fermés par une paroi flexible traversable depuis l'extérieur de l'enceinte pour l'introduction deux bras d'un opérateur en station debout, et un dit plan de travail comprenant des zones délimitées par des rebords périphériques formant des logements creux de formes spécifiques et adaptées pour recevoir et maintenir en place dans chaque dite zone respectivement un matériel ou réactif utile pour la réalisation des dits tests, et un deuxième orifice pour l'évacuation de déchets vers une poubelle, la dite poubelle étant agencée ou apte à être agencée avec la dite enceinte de telle sorte qu'un opérateur puisse évacuer directement dans la dite poubelle, à travers le dit deuxième orifice à l'intérieur de la dite enceinte, un déchet de test tel que du matériel usagé, depuis l'intérieur de l'enceinte que ses mains traversent au niveau des dits premiers orifices de l'enceinte, sans exposer ledit déchet à l'atmosphère extérieure de la dite enceinte et de la dite poubelle, et
- une partie inférieure comprenant ledit mobilier de rangement comprenant la dite armoire réfrigérée indépendante et amovible, et la dite partie inférieure est apte à supporter la dite partie supérieure,
- les dites partie supérieure et partie inférieure de pas plus de 35kg étant portatives manuellement et indépendantes et séparables manuellement l'une de l'autre ; et
- un compartiment de rangement additionnel contenant ou apte à contenir du matériel pour réaliser des prélèvements d'échantillon de fluide ou tissu corporel approprié et/ou du matériel et réactifs utiles pour réaliser les dits test d'analyse microbiologiques, ainsi qu'une poubelle pour déchets biologiques, le dit compartiment de rangement additionnel étant constitué par une zone ouverte en face avant de ladite partie supérieure, située dessous la dite enceinte , entre ladite partie isolée thermique et la dite enceinte, le corps de la dite poubelle étant situé dessous le plan de travail de la dite enceinte.

L'ergonomie de la poubelle résultant de sa position et de son accès directement au niveau du plan de travail dans l'enceinte prévient les risques de renversement de la poubelle et les risques de contaminations qui pourraient en résulter ; ceci est particulièrement important dans le cas où la cabine est embarquée dans un bateau notamment mais aussi dans un camion avec des contraintes d'agitation.

Ces logements creux sont avantageux pour la mise en oeuvre à bord d'un navire en cas de roulis ou tangage. Plus particulièrement, ces logements creux forment des zones de travail dédiées permettant d'éviter les conséquences des mouvements du bateau dans le cas d'une surface totalement plane avec notamment des zones pour porte-tube, zone de déchets, zone pour ustensiles, zone pour réactifs, ainsi qu'une zone au fond équipée d'une prise d'alimentation électrique permettant d'ajouter un automate d'analyses biologiques.

Enfin, on peut ajouter un aimant en façade de l'écran de l'ordinateur permettant d'y fixer un lecteur de bandelettes de tests biologiques pour faciliter la comparaison des résultats négatifs ou positifs avec les résultats positifs et négatifs-types apparaissant sur l'écran de ladite tablette.

La présente invention fournit également une méthode de mise en oeuvre d'un dispositif portatif et/ou mobile comprenant une cabine laboratoire selon l'invention comprenant les dites étapes suivantes dans lesquelles :
- e1) on enregistre à l'aide dudit programme d'ordinateur la sélection du ou les signes ou symptômes ressentis par le patient choisis parmi n signes ou symptômes susceptibles d'être par des patients atteints d'au moins une des m pathologies de chacune des dites k catégories de syndromes de dites maladies, n'étant un nombre entier de préférence au moins égal à k, et
- e2) le dit programme d'ordinateur détermine et indique le ou les types de dites enveloppes contenant la (ou les) trousse(s) de test d'analyse biologique à réaliser en fonction de la dite sélection de l'étape e1), et
- e3) le dit programme d'ordinateur détermine et indique le (ou les) mode(s) opératoire(s) pour effectuer le (ou les) prélèvement(s) d'échantillon(s) biologique(s) de fluide ou sécrétion corporelle dudit patient à analyser à l'aide des matériels et réactifs contenus dans la (ou les) dites(s) enveloppe(s) déterminée(s) à l'étape2) et/ou dans ledit mobilier de laboratoire, et
- e4) le dit programme d'ordinateur détermine et indique le (ou les) mode(s) opératoire(s) pour réaliser et interpréter les résultats des dits test d'analyses biologiques contenus dans la (ou les) dite(s) enveloppes déterminée(s) à l'étape2), et
- e5) on enregistre à l'aide du dit programme d'ordinateur les résultats positif, négatif ou ininterprétable de chacun des dits tests d'analyses biologiques réalisés contenus dans la (ou les) dites(s) enveloppe(s) déterminée(s) à l'étape e2), et
- e6) le dit programme d'ordinateur détermine et indique les actions à entreprendre et/ou conduite à respecter pour le patient et/ou les personnes de son entourage selon les indications fournies par ledit programme d'ordinateur en fonction de la combinaison de résultats positifs ou négatifs ou ininterprétables de tous les tests réalisés enregistrés à l'étape e5).

Plus particulièrement, on réalise les étapes suivantes :
- à l'étape e2), on saisit physiquement dans la dite armoire le (ou les) type(s) de dites enveloppes contenant la ou les trousse de test d'analyse biologique à réaliser qui sont indiquées par ledit programme d'ordinateur, et
- à l'étape e3), on place dans la dite enceinte des matériels et réactifs contenus dans la (ou les) dite(s) enveloppe(s) et/ou dans ledit mobilier de laboratoire selon le (ou les) dit(s) mode(s) opératoire(s) indiqué par ledit programme d'ordinateur pour effectuer au moins un prélèvement d'échantillon biologique de fluide ou sécrétion corporelle dudit patient à analyser, et
- à l'étape e4), on réalise dans l'espace confiné au sein de la dite enceinte le ou les dits tests d'analyses biologiques contenus dans la (ou les) dite(s) enveloppe(s) selon les indications de mode(s) opératoire(s) fournies par ledit programme d'ordinateur, et on évalue les résultats positif, négatif ou ininterprétable de chacun des dits tests d'analyses biologiques réalisés selon les indications fournies par ledit programme d'ordinateur, et
- à l'étape e6) on effectue les actions à entreprendre et/ou conduite à respecter pour le patient et/ou les personnes de son entourage selon les indications fournies par ledit programme d'ordinateur.

Plus particulièrement, la méthode selon l'invention comprend les étapes successives suivantes dans lesquelles :
- e1) ledit programme d'ordinateur affiche sur l'écran de l'ordinateur, n signes ou symptômes pouvant être ressentis par des patients atteints d'au moins une des m pathologies de chacune des dites k catégories de syndromes de dites maladies, n'étant un nombre entier de préférence au moins égal à k, et on sélectionne par contact tactile et/ou u clavier de l'ordinateur les signes cliniques et/ou symptômes ressentis par le patient, et
- e2) le dit programme d'ordinateur affiche sur l'écran de l'ordinateur le ou les types de dites enveloppes contenant la (ou les) trousse(s) de test d'analyse biologique à réaliser, et
- e3) le dit programme d'ordinateur affiche sur l'écran de l'ordinateur le mode opératoire pour effectuer au moins un prélèvement d'échantillon biologique de fluide ou sécrétion corporelle dudit patient à analyser, et
- e4) le dit programme d'ordinateur affiche sur l'écran de l'ordinateur le mode opératoire pour réaliser et interpréter les résultats, et
- e5) le dit programme d'ordinateur affiche sur l'écran de l'ordinateur les instructions pour enregistrer les résultats positif, négatif ou ininterprétable de chacun des dits tests d'analyses biologiques réalisés, et
- e6) le dit programme d'ordinateur affiche sur l'écran de l'ordinateur les actions à entreprendre et/ou conduite à respecter pour le patient et/ou les personnes de son entourage en fonction de la combinaison de résultats positifs ou négatifs ou ininterprétables de tous les tests réalisés.

Plus particulièrement, un panel de signes cliniques et syndromes, est défini, chaque panel requérant la réalisation d'un ou plusieurs tests de diagnostic microbiologique rapide équipant dans des conditions spécifiques ledit laboratoire, les dits tests étant aptes à tester dans un délai inférieur à 1 heure, un certain nombre de microorganismes pathogènes responsables des syndromes cliniques considérés.

Plus particulièrement, les dits tests de diagnostic biologiques rapide fournis et réalisés à l'aide du laboratoire de point-de-soins selon l'invention, peuvent être mis en oeuvre par des professionnels hors santé, ayant reçu une formation spécifique pour la mise en oeuvre d'une méthode aidant pas-à-pas à la réalisation du diagnostic à l'aide de l'équipement spécifique d'un laboratoire compact et mobile selon l'invention.

On entend ici par « syndrome », un symptôme ou signe clinique ou un ensemble de symptômes ou signes cliniques traduisant l'atteinte pathologique affectant un organe ou tissu corporel, notamment un organe ou tissu susceptible d'être infecté et/ou de transmettre une dite infection d'un organe, tissu ou fluide corporel.

On entend par « trousse de test d'analyse biologique », un ensemble de matériels et réactifs nécessaires à la dite analyse microbiologique pour la détection d'un dit microorganisme regroupés dans un même conditionnement aussi appelé communément « kit de diagnostic » ou ci-après « test d'analyse biologique » ou « test de diagnostic ».

On entend ici par « enveloppe » tout type de conditionnement en matériau souple ou rigide. Les dites enveloppes de chaque catégorie d'infection sont aussi appelées ci-après « trousse syndromique ».

Les prélèvements peuvent être des auto-prélèvements et il n'est pas exclu que l'opérateur puisse être le patient lui-même.

De préférence, les microorganismes de chaque enveloppe ou trousse syndromique sont choisis comme étant les microorganismes responsables des infections les plus fréquentes et/ou les plus graves associés audit syndrome en fonction de la zone géographique et/ou de la saison et/ou des et notamment en fonction des épidémies en cours.

Le regroupement des différentes trousses de tests de diagnostic à réaliser pour une dite même catégorie de syndrome de dites maladies au sein d'une dite enveloppe, ainsi que le stockage de différents types d'enveloppes dans différents compartiments distincts de la dite armoire vise à éviter les risques d'erreur de manipulation de l'opérateur dans les conditions d'urgence dans lesquelles ce type de méthode doit être réalisé, surtout lorsque ledit opérateur est ledit patient lui-même.

A l'étape e6), les dites actions à entreprendre choisies parmi au moins :
- évacuation du patient en urgence vers un établissement approprié pour recevoir un traitement thérapeutique et/ou pour faire effectuer des analyses biologiques complémentaires, tel qu'un hôpital, et
- isolement du patient du fait d'une infection contagieuse et/ou mesures d'hygiène pour le patient et/ou les autres personnes de son entourage, et
- communication des résultats des tests d'analyses biologiques à un établissement de traitement à distance pour fourniture d'ordonnance de traitement du patient pour administration sur place et/ou communication d'une proposition de traitement du patient à démarrer sur place.

Les instructions d'évacuation, peuvent dans le cas d'un navire, comprendre des instructions de déroutement du navire.

La dite enceinte constitue une barrière physique de sécurité entre l'opérateur et la zone où des microorganismes pourraient être à son contact.

La méthode selon la présente invention, constitue une aide à la prise de décision très utile pour les centres médicaux de traitements thérapeutiques avec lesquels les navires et notamment les cargos ou les bateaux de croisières ou les sites à terre isolés ou en mer (village de montagne, îles ou plateformes), sont en contact.

La méthode et le dispositif selon l'invention, permettent de faciliter la prise de décision à distance par le médecin du centre médical de traitement ou même en l'absence de possibilité de contact à distance avec un centre de traitement et le cas échéant, permettent d'éviter une évacuation du patient ou un déroutage inutile du navire sur lequel le laboratoire est embarqué en l'absence de contact avec un centre de traitement.

Un autre avantage des méthodes et dispositif selon l'invention est qu'ils peuvent être utilisés par du personnel non qualifié médicalement, tout en assurant de préférence la validation des résultats à distance par des professionnels médicaux.

Plus généralement, les procédés et dispositif selon l'invention assurent un renforcement du service médical sur les navires ou dans les endroits à terre ou en mer isolés et une diminution du coût du diagnostic des infections dans lesdits endroits isolés.

La méthode et le dispositif selon l'invention sont aussi avantageux en ce qu'ils peuvent être mis en oeuvre en assurant la protection de l'opérateur et de l'environnement sans que le matériel et le procédé aient à être mis en oeuvre dans une pièce isolée biologiquement.

Un autre avantage du dispositif selon l'invention et qu'il permet de faciliter le remplacement, des différentes parties du dispositif usagé ou frappé de péremption, et il facilite également la gestion des déchets et la maintenance du matériel.

Plus particulièrement, les méthodes et dispositif selon l'invention permettent d'augmenter la sécurité et la protection du manipulateur et de l'environnement extérieur pour éviter la contamination biologique infectieuse.

Plus particulièrement, ledit mobilier de laboratoire et la dite méthode sont mis en oeuvre à bord d'un navire ou sur une île dépourvue d'établissement d'analyse biologique et/ou dépourvue de traitement médical ou encore dans un site terrestre isolé, notamment en montagne ou désertique.

Dans le mode préféré de réalisation, les m trousses de tests d'analyse biologique de chaque dite catégorie de syndromes de dites maladies, sont regroupés dans une même enveloppe présentant une caractéristique distinctive visuelle différente pour respectivement les différentes dites catégories de syndromes de dites maladies, et les dites enveloppes sont disposés chacune dans un compartiment cloisonné différent de la dite armoire réfrigérée, les dits compartiments cloisonnés présentant des caractéristiques distinctives visuelles différentes entre elles, ledit programme d'ordinateur étant apte à indiquer à l'étape e2) au moins un dit compartiment cloisonné contenant respectivement le dits type d'enveloppe contenant la ou les trousse de tests d'analyses biologiques à réaliser.

Les dits compartiments cloisonnés de l'armoire réfrigérée peuvent être par exemple des tiroirs, et les dits types d'enveloppes et/ou différents zones cloisonnées compartiments cloisonnés peuvent comprendre des moyens de conservation isotherme desdites trousses de test d'analyse biologique.

De préférence encore, les dits tests d'analyse biologique sont réalisables en pas plus de 1heure, de préférence des tests dont les résultats sont révélés sur un support solides présentant une caractéristique visuelle différente selon que le test est positif ou négatif.

Plus particulièrement encore, les tests d'analyses microbiologiques à réaliser permettent une révélation des résultats de manière visuelle et en moins d'une heure et sont du type tests immuno-chromatographiques ou des tests d'analyse d'acides nucléiques impliquant une amplification, de préférence du type PCR ou amplification isotherme.

Plus particulièrement, à l'étape e5), l'évaluation des résultats des dits tests se fait par lecture visuelle de bandelettes de tests.

A l'étape e5), l'opérateur peut enregistrer ou indiquer lui-même les résultats des tests. Toutefois, avantageusement, le programme d'ordinateur interprète automatiquement les résultats bruts des tests d'analyse microbiologique pour déterminer si les tests sont positifs ou négatifs ou ininterprétables, de préférence par lecture visuelle de bandelettes de tests via une caméra ou scanner coopérant avec l'ordinateur.

De préférence encore, chaque syndrome correspond à un signe clinique ou symptôme différent ou dite combinaison différente desdits signes cliniques ou symptômes.

Plus particulièrement, les trousses de tests de d'analyses biologiques comprennent les réactifs et matériels pour réaliser le prélèvement d'un échantillon de fluide tel que urine ou sang ou de sécrétion tel que salive, selles, sécrétions corporelles de muqueuses ou autres organes corporels.

Plus particulièrement, les matériels de prélèvement comprendront :
- des lancettes pour recueil de sang total,
- des écouvillons secs stériles pour recueil de prélèvements urétraux, vaginaux, rectaux, pharyngés ou nasaux, et
- des récipients secs stériles pour recueil des urines.

Plus particulièrement, les différents types d'enveloppes et/ou différents compartiments cloisonnés de ladite armoire, sont identifiés distinctivement visuellement au moins par une couleur différente pour chaque compartiment et/ou chaque type d'enveloppe.

Plus particulièrement, à l'étape 2), ledit programme d'ordinateur indique à l'opérateur un compartiment contenant le ou les ensemble(s) de trousses de tests de diagnostics à réaliser, et un compartiment différent contenant du matériel pour réaliser le prélèvement d'échantillon approprié.

Plus particulièrement, on détermine k=6 catégories de syndromes de dites maladies comprenant un classement des syndromes par le type d'organe pouvant être la cible de ladite infection, comprenant :
- la catégorie des maladies affectant le poumon pour le syndrome « affections respiratoires », et
- la catégorie des infections affectant le larynx ou la gorge pour le syndrome « angine », et
- la catégorie des infections affectant le tube digestif pour le syndrome « gastro entérite », et
- la catégorie des infections affectant les organes sexuels et/ou transmises par contact au niveau des secrétions des organes sexuels pour le syndrome « infections sexuellement transmissibles», et
- la catégorie des infections de maladies tropicales affectant le sang pour le syndrome « fièvre tropicale »,
- la catégorie des infections affectant le tractus urinaire pour le syndrome « infection urinaire », et
- la catégorie des infections affectant le foie pour le syndrome « ictère ».

Plus particulièrement encore, on détermine lesdits microorganismes pathogènes à tester suivants pour les dites catégories d'infections suivantes :
- pour la catégorie des maladies affectant le poumon, les dits microorganismes sont choisis parmi : *Legionella pneumophila* (maladie des légionnaires), *Streptococcus pneumoniae* (pneumocoque), virus *Influenza* (grippe); et virus respiratoire syncitial, et
- pour la catégorie des infections affectant le larynx ou la gorge (angine), les dits microorganismes sont choisis parmi : *Streptococcus pyogenes* (Streptocoque du groupe A) et le virus Epstein Barr (mononucléose infectieuse) ; et
- pour la catégorie des infections affectant le tube digestif, les dits microorganismes sont choisis parmi les Norovirus, Adenovirus rotavirus, *Clostridium difficile, Salmonella enterica Typhi* (typhoide), et *Vibrio cholerae* (choléra); et
- pour la catégorie des infections sexuellement transmissibles affectant les organes sexuels et/ou transmises par contact au niveau des secrétions des organes sexuels, les dits microorganismes sont choisis *Chlamydia trachomatis, Neisseria gonorrhae* (gonococcie), *Treponema pallidum* (syphilis); le virus de l'herpès, et
- pour la catégorie des infections de maladies dites de fièvres tropicales affectant le sang, les dits microorganismes sont choisis parmi : *Plasmodium falciparum* (malaria, paludisme), les bactéries *Borrelia* des fièvres récurrentes, de préférence *Borrelia crocidurae* et *Borrelai duttonii,* le virus de la Dengue et le virus Chikgunuya; *Leptosira interrogans* (Leptospirose) *et Salmonella enterica Typhi* (typhoïde), *et*
- pour la catégorie des infections affectant le tractus urinaire, les dits microorganismes sont choisis parmi : *Escherichia coli* et des bactéries *Proteus* spp., *Citrobacterspp., Enterobacterspp.* et *Klebsiella* spp. Et
- la catégorie des infections affectant le foie pour le syndrome « ictère » ; les dits microorganismes sont choisis parmi : virus de l'hépatite B, virus de l'hépatite C, virus Epstein Barr (mononucléose infectieuse), *Plasmodium falciparum* (malaria, paludisme), *Leptosira interrogans* (Leptospirose) *et Salmonella enterica Typhi* (typhoïde).

Plus particulièrement, on détermine lesdites pathologies non infectieuses suivantes pour la catégorie des maladies affectant le poumon:
- « embolie pulmonaire » pour laquelle on réalise un test de détection rapide par dosage de D-dimères de fibrine dans le sang, et
- « infarctus du myocarde » pour lequel on réalise un test de détection rapide par dosage de troponine.

Ces deux pathologies non infectieuses requièrent les mêmes mesures d'urgence suivantes:
- administration d'anticoagulant tel que l'héparine et oxygénation du patient, et
- la cas échéant évacuation du patient.

Avantageusement encore, on met en oeuvre des trousses complémentaires suivantes :
- trousse pour la détection de tétanos par recherche d'anticorps anti toxine produite par *Clostridium tetani* pour vérifier la protection vaccinale en cas de blessure, et
- des trousses pour la détection de toxiques pour rechercher l'ingestion de drogues telles que: paracétamol, amphétamines, barbituriques, benzodiapénines, cocaïne ; méthadone, opiacés, phéncyclidines, THC, antidépresseurs tricycliques.

Plus particulièrement, encore, les n signes clinique et/ou symptômes comprennent au moins les signes cliniques ou symptômes suivants ou combinaison suivantes:
- mal au ventre, nausées, vomissements et/ou diarrhées, pour le syndrome « gastro entérite »,
- brulure en urinant et/ou fièvre, pour le syndrome « infections urinaires »,
- écoulement ou lésion de l'organe génital, notamment écoulement de la verge, pour le syndrome « infections sexuellement transmissibles»,
- toux et/ou douleur dans le thorax et/ou fièvre pour le syndrome « affections respiratoire »,
- mal à la gorge et/ou fièvre pour le syndrome « angine »,
- fièvre seulement sans autre symptôme ci-dessus, et avec séjour récent en région tropicale pour le syndrome « fièvre tropicale », et
- coloration jaune de la peau pour le syndrome « ictère ».

De préférence, la dite armoire réfrigérée est amovible voire sous forme de casier amovible indépendant comprenant plusieurs dits compartiments.

Le dit casier comprend une pluralité de tiroirs isothermes, ledit casier étant de préférence monté sur et/ou sous la forme d'un chariot mobile de préférence reposant sur des roulettes.

Le dit mobilier de rangement et/ou ladite poubelle peuvent être montés sur des roulettes et/ou la dite armoire réfrigérée peut être également amovible et mobile montée sur des roulettes.

Plus particulièrement, le dispositif selon l'invention comprend en outre des moyens de lecture telle qu'un scanner apte à lire les résultats visuels des dits tests notamment se présentant sous forme de bandelettes et aptes à coopérer avec le dit ordinateur de telle sorte que celui-ci peut interpréter les résultats bruts pour déterminer si les tests sont positifs ou négatifs ou ininterprétables.

Dans une variante, le laboratoire-meuble selon l'invention sera équipé d'un thermocycleur pour la mise en oeuvre de test de diagnostic impliquant des sondes nucléiques et amplifié par PCR ou amplification isotherme.

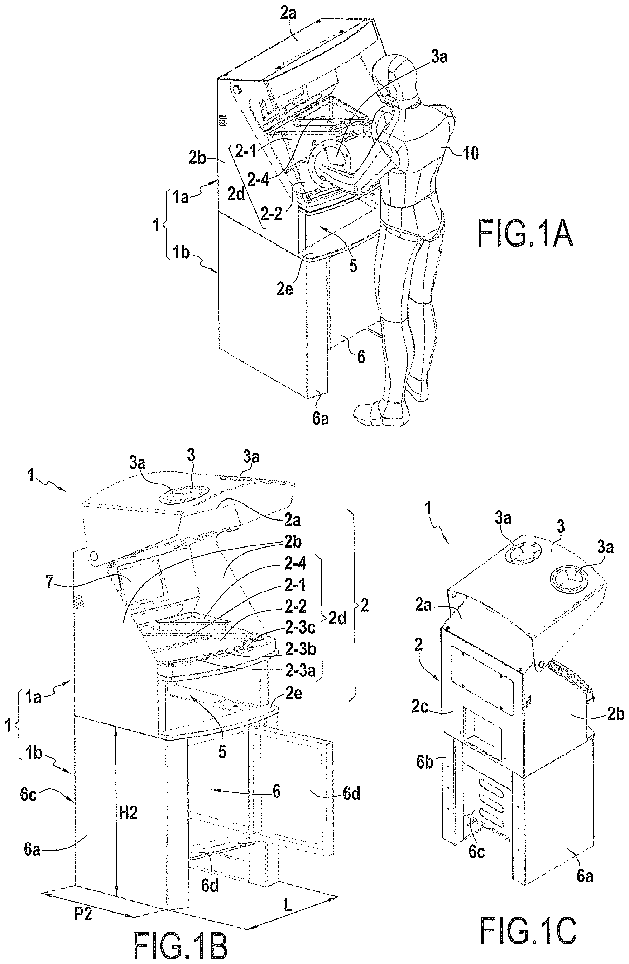

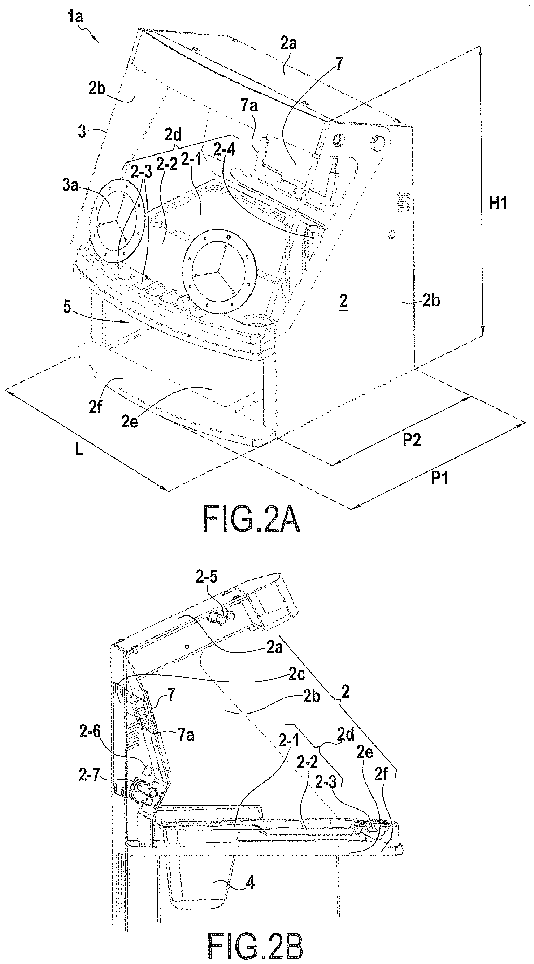

D'autres caractéristiques et avantages de la présente invention apparaitront à la lumière de la description détaillée des exemples qui vont suivre en référence aux figures 1 à 4 dans lesquelles :
- la figure 1A représente un meuble laboratoire compact 1 selon l'invention en cours de mise en oeuvre par un opérateur,
- la figure 1B représente un meuble laboratoire compact selon l'invention avec capot 3 et réfrigérateur 6 ouverts,
- la figure 1C est une vue arrière du meuble laboratoire de la figure 1A,
- la figure 2A représente une vue en perspective la partie supérieure la du laboratoire compact selon l'invention avec le capot fermé,
- la figure 2B représente une vue en coupe verticale de la partie supérieure la du laboratoire compact selon l'invention sans le capot,
- la figure 3 représente une vue en perspective la partie inférieure 1b du laboratoire compact selon l'invention avec le réfrigérateur indépendant pas encore en place sur son étagère 6b, et
- la figure 4 représente la tablette 7 affichant le premier écran sur lequel figurent les signes ou symptômes à sélectionner.

### Exemple 1 : Trousses syndromiques pour laboratoire embarqué en milieu maritime.

Le milieu maritime est un milieu géographiquement isolé. Il n'y a pas de médecin à bord des navires de fret tel que porte containers et le médecin et l'infirmerie des navires de croisière ne dispose pas actuellement de moyens de diagnostics rapides des infections graves et contagieuses, obligeant par précaution à des déroutements parfois inutiles et couteux.

Dans cet exemple, les inventeurs ont déterminé six enveloppes ou trousses syndromiques de couleurs différentes facilement reconnaissables visuellement, comportant une trousse « Angine », une trousse « Infection urinaire », une trousse « Diarrhée », une trousse « Infection respiratoire », une trousse « Fièvre tropicale » et une trousse « Infections Sexuellement Transmissibles (IST) » ou « kit MST ».

Chacune de ses trousses (aussi dénommée ci-après « kit ») est destinée à être utilisée en fonction des signes ou combinaisons de signes cliniques suivants :
- trousse « Angine » en cas de « mal à la gorge » et/ou fièvre au moins,
- trousse « Infection urinaire » en cas de « Brûlures en urinant » et/ou fièvre ,
- trousse « Gastro entérite » (ou ci-après « kit digestif ») en cas de « mal au ventre » et/ou « nausées » et/ou « vomissements » et/ou « diarrhée » au moins,
- trousse « affection respiratoire » (ou ci-après « kit pulmonaire ») en cas de « Douleur dans le thorax » et/ou « toux » et/ou fièvre,
- trousse « Infections Sexuellement Transmissibles » en cas de « Ecoulement ou lésion de l'appareil génital », notamment «écoulement de la verge », et
- trousse « affection hépatique » (ci-après « kit ictère ») en cas coloration jaune de la peau, et
- trousse « Fièvre tropicale » en cas de « fièvre » (sans autre symptôme ci-dessus) et séjour récent en région tropicale, notamment en Afrique.

Si la fièvre n'est pas associée à au moins un autre symptôme, et que le patient n'a pas séjourné en région tropicale, les « kit Angine » « kit pulmonaire » et« kit Infection urinaire » sont à mettre en oeuvre.

Si la fièvre est associée à au moins un autre symptôme, ce(s) dernier(s) symptôme(s) détermine(nt) le kit à mettre en oeuvre.

Si la toux est associée à un autre symptôme, ce(s) dernier(s) symptôme(s) détermine(nt) le kit à mettre en oeuvre.

Si le symptôme « brulure en urinant » est associé à un autre symptôme (écoulement de la verge), ce dernier symptôme sera déterminant pour mettre en oeuvre le « kit MST ».

Les 7 trousses syndromiques comportent des kits de tests d'analyse microbiologiques ci-après dénommés « test de diagnostic ». Ces tests impliquent la technique dite d'immuno chromatographie (ICT) et sont disponibles dans le commerce. Ils impliquent la mise en oeuvre de supports solides avec une bandelette sur laquelle est fixée un anticorps ou un substrat d'enzyme spécifique d'un microorganisme pathogène et une révélation colorée sur la bandelette d'une réaction de l'échantillon de prélèvement testé. Ces tests immuno chromatographiques dont le résultat se traduit par une coloration sont simples et facile d'interprétation par un personnel non médical.

Les différents tests de diagnostic de chaque trousse syndromique sont conditionnés dans des boites identifiées par une lettre différente et de même couleur que celle de l'enveloppe de la trousse syndromique.

Les tests seront choisis pour chaque syndrome en fonction de l'épidémiologie des infections concernée par la localisation du port d'origine et/ou du trajet du navire, de la ou les saisons durant le trajet et des épidémies en cours.

A titre illustratif, la composition des 7 trousses sera la suivante.

### 1) la trousse Angine comporte:

(a) un écouvillon (Greiner Bio One, Courtaboeuf, France) pour prélèvement pharyngé,
(b) une lancette (MediPurpose, Duluth, USA) pour prélèvement sanguin;
(c) un test de diagnostic rapide immuno chromatographique (ICT) de détection de Streptococcus groupe A, Streptococcus pyogenes A (fournisseur : société AESD, Berlin, Allemagne) pour la détection directe du Streptococcus groupe A sur écouvillon pharyngé (ci-après « test strepto A»),
(d) un test de diagnostic rapide de détection d'anticorps du virus Epstein-Barr sur prélèvement sanguin (société AESD, Berlin, Allemagne),

Ces deux infections représentent environ 70% des infections correspondant à ce syndrome soit environ 60% pour le Streptococcus groupe A et 10% pour le virus Epstein-Barr.

Les résultats obtenus dans chacun de ces tests orientent la conduite médicale comme suit :
- si le « test strepto A» est positif : ordre de communication du résultat à un centre médical habilité à distance pour prescription d'un traitement à l'aide d'antibiotique (pénicilline), et
- si le « test virus Epstein-Barr » est positif : ordre de communication du résultat à un centre médical à distance pour prescription d'un traitement des symptômes sans antibiotique et recommandation de repos.

Dans les deux cas aucun isolement ni transfert ou déroutement du navire d'urgence ne sont requis.

### 2) la trousse « Infection urinaire » (aussi dénommé « kit urine ») comporte :

(a) un pot à urines (Dominique Dutscher, Brumath, France), et
(b) bandelette urinaire (*Mission*@UTI Test Strips, Acon Laboratories, San Diego, USA).

Ces bandelettes urinaires sont revêtues de :
- substrats d'enzymes nitrate-réductases spécifiques des bactéries *E. coli* impliquées dans 80% des infections urinaires et bactéries *Proteus mirabilis ou autres Enterobactéries,* (ci-après « test nitrites ») et
- substrats d'enzymes estérases spécifiques des globules polynucléaires neutrophiles présents dans tous les cas d'infections urinaires (ci-après « test leucocytes »).

Ces bandelettes urinaires que l'on trempe dans l'échantillon d'urines prélevées dans le pot à urines, réagissent de façon colorée en cas d'infection urinaire.

Le résultat obtenu dans ce test oriente la conduite médicale comme suit :
- si les « tests nitrites » et « tests leucocytes » sont tous les 2 négatifs pas de traitement, et
- si l'un des 2 tests est positif : ordre de communication du résultat à un centre médical habilité à distance pour prescription d'un traitement à l'aide d'antibiotique.

Aucun isolement ni transfert ou déroutement du navire d'urgence ne sont requis.

### 3) la trousse « gastro entérite » (ou « kit digestif ») comporte :

(a) pot à coproculture (CML, Nemours, France) pour prélèvement de selles pour les tests (c) à (e) ci-après, et
(b) une lancette (MediPurpose, Duluth, USA) pour prélèvement sanguin pour les tests (f) et (g) ci-après.
(c) test immunologique de détection rapide de Norovirus par ICT (RIDA@QUICK NOROVIRUS, R-BIOPHARM AG, Darmstadt, Allemagne), et
(d) test immunologique de détection rapide de Adeno-rotavirus par ICT (AESD, Berlin Allemagne).

Ces types d'infections virales représentent environ 70% des infections correspondant à ce syndrome.

Le résultat obtenu dans ces tests oriente la conduite médicale comme suit : si l'un des tests est positif :
- instructions d'isolement de la personne pendant au moins 48 heures après la cessation des diarrhées, une chambre et des toilettes doivent être dédiées à la personne uniquement et usage de couverts à usage unique pour les repas, et
- conseils d'hygiène des locaux par nettoyage à l'eau de javel et de l'entourage par lavage des mains avec savon et solution hydroalcoolique, et
- instructions de communication du résultat à un centre médical habilité à distance pour prescription d'un traitement des symptômes.
   (e) test immunologique de détection rapide de Adénovirus et des rotavirus par ICT (AESD, Berlin, Allemagne).
   (f) test de détection de *Vibrio cholerae* par ICT (AESD, Berlin, Allemagne) (choléra).
   (g) test de détection de *Salmonella enterica Typhi* par ICT (AESD, Berlin, Allemagne) (fièvre typhoïde).

### 4) La trousse « affection respiratoire » (ou « kit pulmonaire ») comporte:

(a) écouvillon pharyngé ou nasal (Greiner Bio One) pour les tests (d) et (e) ci-après,
(b) pot à urines (Dominique Dutscher) pour les tests (f) et (g) ci-après,
(c) une lancette (MediPurpose, Duluth, USA) pour prélèvement sanguin pour les tests (f) et (g) ci-après.
(d) test immunologique de détection du type ICT du virus de la grippe (virus *influenza*) (AESD, Berlin, Allemagne) pour détection directe sur écouvillon pharyngé,
(e) test de détection du virus respiratoire syntitial (VRS) par ICT (AESD, Berlin, Allemagne).
(f) un test immunologique du type ICT de diagnostic rapide de *Legionella pneumophila* (SLIDEX@Legionella Kit, bioMérieux, France) pour détection directe de *Legionella pneumophila* sur urines (ci-après « test légionella »),
(g) un test immunologique du type ICT de diagnostic rapide de *Streptococcus pneumoniae* (SLIDEX@Pneumo Kit, bioMérieux, France) pour détection directe de *Streptococcus pneumoniae* sur urines (ci-après « test pneumo »).

En saison épidémique, la grippe représente environ 80% des infections correspondant à ce syndrome tandis que les deux autres infections représentent environ 5% chacune en toute saison et présentent des risques mortels.

Les résultats obtenus dans chacun de ces tests orientent la conduite médicale comme suit :
- si un test de la grippe et/ou VRS est positif : isolement de la personne, instructions de communication des résultats à un centre médical habilité à distance pour prescription d'un traitement des symptômes notamment à l'aide d'antibiotique (de la famille des pénicillines) pour la grippe.
- si le « test Legionella » est positif : isolement de la personne non requis, instructions de communication des résultats à un centre médical habilité à distance pour prescription d'un traitement des symptômes et à l'aide d'antibiotique (de la famille des macrolides ou des cyclines).
- si le « test pneumo » est positif : isolement de la personne requis et instructions de communication des résultats à un centre médical habilité à distance pour prescription d'un traitement des symptômes et à l'aide d'antibiotique (de la famille des pénicillines) voire déroutement du navire pour soins plus intenses.

D'autres virus peuvent être recherchés selon les régions ou saisons concernées notamment le pneumovirus et rhinovirus.

Avantageusement, quels que soient les résultats des tests (d) à (g), on teste en outre, lesdites pathologies non infectieuses suivantes pour la catégorie des maladies affectant le poumon:
(h)- « embolie pulmonaire » pour laquelle on réalise un test de détection rapide par dosage de D-dimères de fibrines dans le sang (Triage@D-Dimer, Alère, France), et
(i)- « infarctus du myocarde » pour lequel on réalise un test de détection rapide par dosage de troponine (Triage@Troponine, Alère, France).

Ces deux pathologies non infectieuses requièrent les mêmes mesures d'urgence si l'un des test est positif:
- administration d'anticoagulant tel que l'héparine et oxygénation du patient, et
- la cas échéant évacuation du patient.

### 5) La trousse Fièvre tropicale ou « kit fièvre tropicale » comporte:

(a) une lancette pour prélèvement sanguin pour tous les tests,
(b) un test immunologique de type ICT de diagnostic rapide du Paludisme pour détection directe de *Plasmodium falciparum* sur échantillon de sang (VIKIA® Malaria Ag Pf/Pan, bioMérieux France) (ci-après « test paludisme »),
(c) un test immunologique de type ICT de diagnostic rapide du virus de la dengue (AESD, Berlin, Allemagne) pour détection directe sur sang (ci-après « test dengue »).
d) un test immunologique de type ICT de diagnostic rapide du virus chickungunya (AESD, Berlin, Allemagne) pour détection directe sur sang (ci-après « test chickungunya »).

Dans les régions tropicales ce type d'infections virales représente environ 60% des infections correspondant à ce syndrome, à savoir 30% pour le paludisme et 30% pour le virus de la dengue ou chickungunya.
e) un test immunologique de type ICT de diagnostic rapide de *Leptospira interrogans* (AESD, Berlin, Allemagne) pour détection directe sur sang (ci-après « test leptospirose »).
f) un test immunologique de type ICT de diagnostic rapide de *Salmonella enterica* Typhi (AESD, Berlin, Allemagne) pour détection directe sur sang (ci-après « test typhoïde »).

Les résultats obtenus dans chacun de ces tests orientent la conduite médicale comme suit :
- si le « test paludisme » est positif : isolement de la personne, instructions de ne pas donner d'agent anti-inflammatoire, et instructions de communication urgente des résultats à un centre médical habilité à distance pour prescription d'un traitement des symptômes et à l'aide d'un agent antiparasitaire (de la famille de la chloroquine) voire déroutement du navire pour soins plus intenses.
- si le « test dengue » ou « test chickungunya » est positif : isolement de la personne non requis, instructions de ne pas donner de médicament anti-inflammatoire, et instructions de communication des résultats à un centre médical habilité à distance pour prescription d'un traitement des symptômes sans antibiotique.
- si le « test leptospirose » ou « test typhoïde » est positif : isolement de la personne non requis, et instructions de communication des résultats à un centre médical habilité à distance pour prescription d'un traitement des symptômes avec antibiotique.

### 6) la trousse « Infections Sexuellement Transmissibles » ou « kit MST » comporte:

(a) écouvillons secs stériles pour recueil de prélèvements sur lésions urétraux ou vaginaux et pour écoulement de la verge (Greiner Bio One),
(b) pot à urines (Dominique Dutscher) pour les tests (d) et e)ci-après,
(c) un test immunologique de type ICT de diagnostic rapide du virus de l'Herpes (StrongStep® HSV 1/2, LIMING BIO, Nanjing, Chine) pour détection directe sur écouvillon,
(d) tests immunologique de type ICT de diagnostic rapide de *Chlamydia trachomatis,* (StrongStep®Chlamydia trachomatis, LIMING BIO, Nanjing, Chine) pour détection directe sur urines (ci-après « test chlamydia »),
(e) tests immunologique de type ICT de diagnostic rapide *de Neisseria gonorrhae* (StrongStep® Neisseria gonorrhae, LIMING BIO, Nanjing, Chine) pour détection directe sur urines (ci-après « test gonocoque »).
(f) (e) tests immunologique de type ICT de diagnostic rapide *de Treponema pallidum;* (AESD,Berlin, Allemagne) pour détection directe sur urines (ci-après « test syphilis »).

Les infections d) et e) représentent environ 80% des infections correspondant à ce syndrome tandis que l'infection virale ce) (herpes) représente environ 5% et l'infection f) (syphilis) représente environ 10%.

Les résultats obtenus dans chacun de ces tests orientent la conduite médicale comme suit : si un des tests est positif : instructions de communication des résultats à un centre médical habilité à distance pour prescription d'un traitement antiviral pour l'herpès et traitement antibiotique (tétracycline comme la doxycycline) pour les deux tests selon et conseils d'hygiène et de de prévention (préservatifs).

### 7) La trousse « affection hépatique » ou « kit ictère » comporte :

(a) une lancette pour prélèvement sanguin pour tous les tests,
(b) un test immunologique de type ICT de diagnostic rapide du Paludisme pour détection directe de *Plasmodium falciparum* sur échantillon de sang (VIKIA® Malaria Ag Pf/Pan, bioMérieux France) (ci-après « test paludisme »),
(c) un test de diagnostic rapide de détection d'anticorps du virus Epstein-Barr sur prélèvement sanguin (société AESD, Berlin, Allemagne),
(d) un test immunologique de type ICT de diagnostic rapide de *Leptospira interrogans* (AESD, Berlin, Allemagne) pour détection directe sur sang (ci-après « test leptospirose »),
(e) un test immunologique de type ICT de diagnostic rapide de *Salmonella enterica typhi* (AESD, Berlin, Allemagne) pour détection directe sur sang (ci-après « test typhoïde »).
(f) un test de diagnostic rapide de détection d'anticorps du virus de l'hépatite B sur prélèvement sanguin (société AESD, Berlin, Allemagne),
(g) un test de diagnostic rapide de détection d'anticorps du virus de l'hépatite C sur prélèvement sanguin (société AESD, Berlin, Allemagne).
   - si le « test paludisme » est positif : isolement de la personne, instructions de ne pas donner d'agent anti-inflammatoire, et instructions de communication urgente des résultats à un centre médical habilité à distance pour prescription d'un traitement des symptômes et à l'aide d'un agent antiparasitaire (de la famille de la chloroquine) voire déroutement du navire pour soins plus intenses.
   - si le « test virus Epstein-Barr » ou l'un des tests hépatite B ou C est positif : ordre de communication du résultat à un centre médical à distance pour prescription d'un traitement des symptômes sans antibiotique et recommandation de repos.
   - si le « test leptospirose » ou « test typhoïde » est positif : isolement de la personne non requis, et instructions de communication des résultats à un centre médical habilité à distance pour prescription d'un traitement des symptômes avec antibiotique.

Dans tous les tests des 7 trousses ci-dessus : si tous les résultats de tous les tests sont négatifs ou ininterprétables, aucune recommandation d'action n'est fournie.

Le laboratoire est aussi équipé des consommables suivants communs utiles aux six trousses syndromiques :
- des compresses stériles imprégnées d'antiseptique (H&W, Glabbeek, Belgique),
- une solution hydro-alcoolique (Anios, Lille-Hellemmes, France),
- des gants non-stériles et jetables (Sempermed, vienne, Autriche),
- un container pour déchets bio-hazard (APmédical a.b.m., Erstein, France), et
- des sacs poubelles de 15 litres (Paredes, Rousset, France).

### Exemple 2 : Laboratoire miniaturisé selon l'invention.

Il s'agit d'un laboratoire compact d'analyse sous forme de caisson, cabine ou borne 1 composée de deux ou trois parties portatives indépendantes et/ou séparables de dimension limitée et ne pesant pas plus de 30kg chacune de façon à pouvoir être transportée par une personne seule et/ou en faciliter le transport aérien en soute à bagages le cas échéant.

Ces deux ou trois parties sont les suivantes :
(1) une partie supérieure la constituant le poste de travail reposant sur la partie inférieure 1b, comportant :
   (a) une enceinte de confinement 2 du type boîte à gants délimitée par un toit incliné à une pente 2a, deux parois latérales verticales parallèles 2b reposant sur un plateau support inférieur 2e et une paroi arrière 2c assurant la jonction entre les bords arrières dudit toit 2a, des parois latérales 2b et du plateau inférieur 2e ; un plan de travail horizontal 2d formant le plancher de la dite enceinte, la face avant de ladite enceinte étant fermée par un capot transparent pivotant 3 comprenant deux ouvertures circulaires 3a obturées et aptes à permettre l'introduction des mains et bras de l'opérateur 10, ledit plan de travail horizontal 2d étant disposé en hauteur au-dessus du dit plateau inférieur 2e, et
   (b) une zone de rangement 5 ouverte sur l'avant, délimitée par le plancher 2d de l'enceinte, les parties inférieures des parois latérales 2b et paroi arrière 2c de l'enceinte, et le plateau inférieur 2e, une tablette 2f s'étendant en prolongation vers l'avant du plateau inférieur 2e, et
(2) une partie inférieure 1b sur laquelle repose le plateau support inférieur 2e de ladite partie supérieure ladite partie inférieure 1b comportant deux parois latérales parallèles verticales formant deux piètements 6a reliés par une paroi transversale arrière verticale 6c, une étagère horizontale 6b formant une armoire réfrigérateur 6 apte à être fermée par une porte sur l'avant 6d ou une armoire apte à contenir un réfrigérateur 6 indépendant apte à être posé sur la dite étagère 6b, le dit réfrigérateur 6 permettant le stockage des trousses syndromiques.

Les deux ouvertures circulaires 3a du capot 3 sont obturées une paroi en matière plastique percée par une découpe centrale en forme de Y définissant ainsi des éléments de paroi flexibles juxtaposés aptes à permettre l'introduction des mains et bras de l'opérateur à travers la fente en Y. Ces ouvertures peuvent être complétées par des manchons aptes couvrir l'avant-bras de l'opérateur à l'intérieur de l'enceinte.

Le plan de travail qui constitue le plancher 2d de l'enceinte confinée, comporte une ouverture triangulaire 2-4 supportant et donnant accès à une poubelle 4 dans la zone de rangement 5, la poubelle 4 est équipée d'un sac poubelle d'un volume de 15 litres pour déchets non-contendants facilitant leur élimination depuis la dite enceinte.

Le plancher ou plan de travail 2d de l'enceinte 2 comporte les zones suivantes :
- une zone plane arrière surélevée 2-1 au niveau de laquelle l'opérateur réalise les tests de diagnostic et au niveau de laquelle se trouve l'ouverture 2d de la poubelle 8,
- une zone avant plane 2-2 au niveau de laquelle l'opérateur pose et ouvre le récipient de prélèvement ainsi que certains réactifs et/ou matériels contenus dans la trousse syndromique en cours d'utilisation, et
- au niveau de la bord avant, des compartiments 2-3 délimités par des rebords en saillie formant des logements creux aptes à recevoir : pour le compartiment 2-3a une pipette, pour les compartiments 2-3b des flacons de réactifs contenus dans les conditionnements des dits tests de diagnostic, et pour le compartiment 2-3c, des embouts de pipettes, et
- un trou latéral triangulaire 2-4 apte à recevoir la poubelle 4, celle-ci étant contenue dans la zone de rangement 5 dessus la dite ouverture triangulaire.

Le toit incliné 2a supporte en sous face des lampes UV 2-5 de décontamination au-dessus du plan de travail.

La paroi de fond 2c de l'enceinte supporte une prise électrique 2-6 une lampe 2-7 d'éclairage du plan de travail, un support 7a de tablette ordinateur amovible 7 et sur l'arrière de la paroi arrière, un dispositif de branchement (non représenté) des câbles d'alimentation et prises électriques du réfrigérateur, de la tablette et des lampes UV et lampe d'éclairage.

La tablette 7 est équipée d'un logiciel d'aide à la mise en oeuvre de la méthode selon l'invention qui a comme fonctions principales explicitées ci-après, d'enregistrer les syndromes du patient, de les convertir en une proposition de trousse diagnostique, de donner à l'opérateur toutes les indications nécessaires et suffisantes pour la réalisation des prélèvements cliniques, pour la réalisation des tests diagnostiques contenus dans les trousses syndromiques et pour leur interprétation ainsi que pour le pilotage spécifique des opérations de déroulement de la méthodologie décrite à l'exemple 3.

La cabine 1 est constituée par des matériaux répondant à plusieurs critères de solidité mécanique, de résistance aux variations de température, d'hygrométrie et de salinité ; et de résistance aux produits décontaminant.

La hauteur du meuble laboratoire décrit ci-dessus est telle qu'un opérateur en position debout peut insérer les mains dans les ouvertures 3 et manipuler les réactifs et matériels sur le plan de travail 2d.

Les caractéristiques dimensionnelles maximales d'une borne-laboratoire portatif tel que décrit ci-dessus sont :
- partie supérieure 1a: L=67.5cm x H1=86.6cm x P1=62cm, et
- partie inférieure 1b : L=67.5cm x H2=74.5cm x P2=57.6cm.

Les trousses syndromiques de couleurs différentes pour les 6 catégories de trousses sont rangées dans le réfrigérateur 6.

La zone de rangement 5 ouverte sur l'avant permet de ranger à température ambiante les consommables communs utiles aux six trousses syndromiques mentionnés à l'exemple 1 comprenant des compresses stériles imprégnées d'antiseptique, une solution hydro-alcoolique, des gants non-stériles et jetables, et des sacs poubelles de 15 litres.

Dans une variante, le dispositif peut comprendre un scanner et/ou une caméra coopérant avec la tablette et permettant de numériser les résultats des tests de diagnostic réalisés et les transmettre vers un ordinateur éventuellement situé à distance, notamment un centre thérapeutique à terre.

Dans une variante non représentée, la partie inférieure la est munie de roulettes autobloquantes. Dans une autre variante, le réfrigérateur 6 repose au sol et est muni de roulettes autobloquantes et le réfrigérateur comporte des tiroirs de différentes couleurs contenant chacun une même catégorie de trousses syndromique.

### Exemple 3 : Mode opératoire.

Un opérateur 10, notamment le lieutenant de pharmacie du navire ou le cas échant le médecin de bord ou toute autre personne non médicale, y compris le patient lui-même, utilisera le laboratoire portatif de l'exemple 2 pour la mise en oeuvre de l'ensemble de trousses syndromiques de l'exemple 1 comme suit.

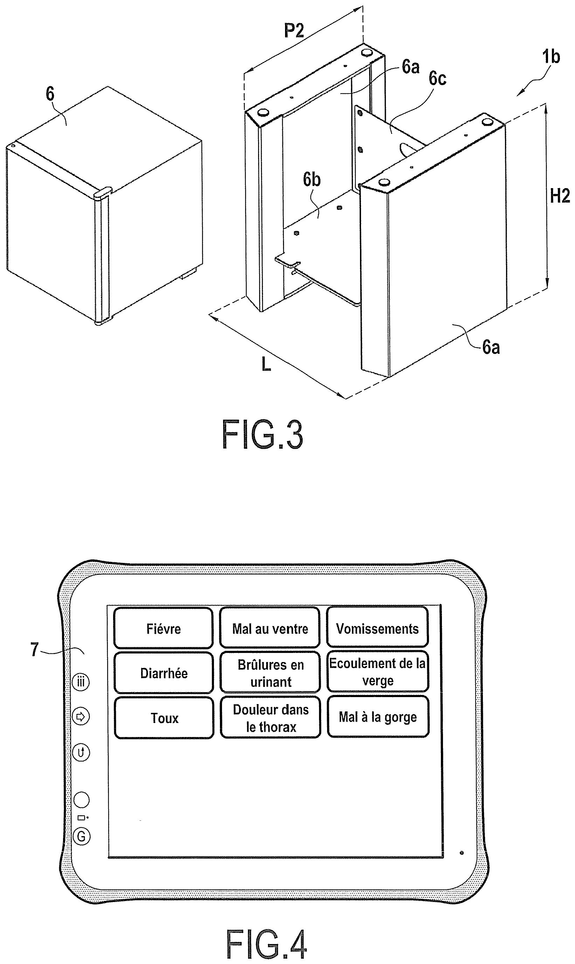

L'opérateur et le patient effectuent les étapes suivantes.
1) l'opérateur ouvre le capot, ouvre l'alimentation de la tablette 7 et sort la tablette de son support pour activer le logiciel de guide et utilisation de réalisation de la méthode selon l'invention avec le dispositif selon l'invention et suivre les instructions du logiciel.
2) Le logiciel affiche un premier écran (figure 4) sur lequel figurant les signes ou symptômes qui peuvent être sélectionnés par le patient.
3) Le logiciel affiche un deuxième écran sur lequel figure la trousse syndromique correspondant au(x) symptôme(s) sélectionnés par le patient, codés par l'une des six couleurs.
4) l'opérateur ouvre le réfrigérateur 6 et sort une trousse syndromique dont l'enveloppe est de la couleur indiquée et la pose sur la zone sur élevée 2-1 du plan de travail 2c, ouvre l'enveloppe et sort les réactifs et matériel contenus, qu'il range dans sur la zone de travail 2-2 et compartiment 2-3 du plan de travail.
5) Le logiciel affiche au moins un troisième écran sur lequel figure les instructions pour la réalisation du prélèvement d'échantillon biologique de selle, urine, sang ou sécrétion à analyser le cas échéant par auto prélèvement par le patient. Selon les instructions affichées :
   - l'opérateur donne au patient le matériel concerné par le type de prélèvement concerné ;
   - Le patient effectue le prélèvement qu'il conditionne dans un récipient hermétiquement clos ;
   - l'opérateur pose le prélèvement clos sur la zone de travail 2-2 et referme le capot 4 de l'enceinte.
   A titre illustratif, dans l'exemple d'une trousse « Infection urinaire », la tablette affiche comme instruction de sortir le test bandelette urinaire de la trousse « Infection urinaire »
6) Le logiciel affiche au moins un quatrième écran sur lequel figure les instructions pour la réalisation et l'interprétation des résultats du ou des tests d'analyse biologiques contenus dans la trousse syndromique.
   A titre illustratif, dans l'exemple d'une trousse « Angine », la tablette donne comme instruction de déposer une goutte de sang sur la bandelette AESD Epstein-Barr Virus correctement orientée, prélevée par la pipette à partir de la lancette et d'attendre 5 minutes.
   Au cours de ces instructions, l'usage de la pipette contenu dans un compartiment 2-3a nécessitera le remplacement de son embout, à l'aide d'embouts stockés dans le compartiment 2-3c et évacuation dans la poubelle après chaque usage.
7) Le logiciel affiche au moins un cinquième écran sur lequel figure les instructions pour enregistrer les résultats des tests de diagnostics avec trois options : test positif, test négatif et test ininterprétable.
   A titre illustratif, dans l'exemple d'une trousse « Gastro-entérite », la tablette donne comme instruction de rechercher un bande grise ou noire en regard du témoin positif ; de conclure à un test ininterprétable si cette bande est absente ; de poursuivre la lecture de la bande en regard de l'échantillon et de conclure à un test négatif si cette bande est absente et à un test positif si cette bande est présente.
8) Le logiciel affiche au moins un sixième écran sur lequel figure le diagnostic éventuel et des instructions concernant la conduite à tenir (transmission des résultats et/ou proposition de traitement thérapeutique à un centre de traitement thérapeutique à distance, isolement du patient, conseils d'hygiènes du patient et des autres personnes à bord, déroutement du navire) en fonctions des résultats sélectionnés des tests de diagnostics.

A titre illustratif, dans l'exemple d'une trousse « Fièvre tropicale » et d'un test interprété positif pour le paludisme à l'étape 5, la tablette donne comme instruction de contacter sans délai le centre médical approprié pour obtenir une conduite à tenir médicale.

## Revendications

1. Dispositif de laboratoire utile pour la mise en oeuvre d'une méthode de diagnostic comprenant la réalisation d'une série de tests d'analyses biologiques in vitro pour le diagnostic de maladies aiguës requérant une intervention médicale urgente comprenant au moins une pluralité de maladies infectieuses aiguës comprenant :
- k types d'enveloppes contenant chacune m trousses de tests d'analyse biologique, les dites m trousses de tests d'analyses biologiques permettant la détection chacune de respectivement m pathologies de k catégories de syndromes de dites maladies aiguës comprenant au moins des catégories de syndromes d'infections par des microorganismes pathologiques, les dites catégories de syndromes de dites maladies étant associées chacune à un syndrome différent affectant un organe ou tissu corporel, k étant un nombre entier supérieur à 2, de préférence k étant au moins égal à 6 catégories, m étant un nombre entier de préférence de 1 à 5, m pouvant être différent pour chaque dite catégorie de syndrome de dite maladie ,
- une enceinte (2) équipée d'un plan de travail (2d), au sein de laquelle enceinte on peut réaliser les dits tests d'analyses biologiques in vitro, et
- un mobilier de rangement (5,1b) comprenant au moins une armoire isolée thermiquement (6), ladite armoire contenant ou apte à contenir une pluralité de dites enveloppes contenant chacune au moins une dite trousse de test d'analyse biologique, le dit mobilier de rangement (5,1b) étant disposé sous de ladite enceinte,
Le dispositif étant **caractérisé en ce qu'**il se présente sous forme d'une cabine portative et/ou mobile, transportable manuellement par une personne seule, comprenant :
- une enceinte (2) fermée en face avant par un capot transparent (3) amovible ou pivotant, munie d'au moins une ouverture d'accès (3a) pour les deux bras d'un opérateur (10), au sein de laquelle enceinte un opérateur peut réaliser les dits tests d'analyse biologique in vitro sur ledit plan de travail en position debout, dans un espace confiné avec ledit capot, et
- un ordinateur (7), de préférence sous forme de tablette, disposé au sein de la dite enceinte au-dessus dudit plan de travail, équipé d'un programme apte à assister l'opérateur pour la réalisation des dits tests d'analyses biologiques ou d'un support d'enregistrement lisible par ledit ordinateur sur lequel est enregistré un dit programme d'ordinateur; le dit programme d'ordinateur étant configuré pour l'exécution d'une série d'étapes guidant le choix et la réalisation ainsi que l'interprétation des résultats desdits tests d'analyses biologiques et la détermination les actions à entreprendre et/ou conduite à respecter pour le patient et/ou le personnel de son entourage en fonction des résultats des tests, comprenant au moins la succession des dites étapes suivantes:
- e1) l'enregistrement de la sélection de signes cliniques et/ou symptômes ressentis par le patient choisis parmi n signes ou symptômes enregistrés dans ledit programme d'ordinateur, pouvant être ressentis par des patients atteints d'au moins une des m pathologies de chacune des dites k catégories de syndromes de dites maladies, n'étant un nombre entier de préférence au moins égal à k, et
- e2) la détermination et l'indication du ou des types de dites enveloppes contenant la (ou les) trousse(s) de test d'analyse biologique à réaliser en fonction de la dite sélection de l'étape e1), et
- e3) la détermination et l'indication du ou des modes opératoires pour effectuer au moins un prélèvement d'échantillon biologique de fluide ou sécrétion corporelle dudit patient à analyser, à l'aide des matériels et réactifs contenus dans la (ou les) dites(s) enveloppe(s) déterminée(s) à l'étape2) et/ou dans ledit mobilier de laboratoire, et
- e4) la détermination et l'indication du ou des modes opératoires pour réaliser et interpréter les résultats desdits tests d'analyses biologiques contenus dans la (ou les) dites(s) enveloppe(s) déterminée(s) à l'étape e2), et
- e5) l'enregistrement du résultat positif, négatif ou ininterprétable de chacun des dits tests d'analyses biologiques réalisés contenus dans la (ou les) dites(s) enveloppe(s) déterminée(s) à l'étape e2), et
- e6) la détermination et l'indication des actions à entreprendre et/ou conduite à respecter pour le patient et/ou les personnes de son entourage en fonction de la combinaison de résultats positifs ou négatifs ou ininterprétables de tous les tests réalisés enregistrés à l'étape e5).

2. Dispositif selon la revendication 1 **caractérisé en ce que** les dits enregistrements et dites indications des étapes e1) à e6) sont opérées ou fournies via une interface graphique de l'écran dudit ordinateur avec laquelle ledit opérateur peut interagir par contact tactile et/ou via un clavier.

3. Dispositif selon la revendication 1 ou 2 **caractérisé en ce que** la dite cabine laboratoire comprend :
- une partie supérieure (1a) comprenant la dite enceinte (2) avec un dit capot (3) munie de deux premiers orifices circulaires (3a) fermés par une paroi flexible traversable depuis l'extérieur de l'enceinte pour l'introduction deux bras d'un opérateur en station debout, et un dit plan de travail (2d) comprenant des zones délimitées par des rebords périphériques formant des logements creux (2-3) de formes spécifiques et adaptées pour recevoir et maintenir en place dans chaque dite zone respectivement un matériel ou réactif utile pour la réalisation des dits tests, et un deuxième orifice (2-4) pour l'évacuation de déchets vers une poubelle, la dite poubelle (4) étant agencée ou apte à être agencée avec la dite enceinte de telle sorte qu'un opérateur puisse évacuer directement dans la dite poubelle, à travers le dit deuxième orifice à l'intérieur de la dite enceinte, un déchet de test tel que du matériel usagé, depuis l'intérieur de l'enceinte que ses mains traversent au niveau des dits premiers orifices de la enceinte, sans exposer ledit déchet à l'atmosphère extérieure de la dite enceinte et de la dite poubelle, et
- une partie inférieure (1b) comprenant ledit mobilier de rangement (1b) comprenant la dite armoire réfrigérée (6) indépendante et amovible, et la dite partie inférieure est apte à supporter la dite partie supérieure,
- les dites partie supérieure (1a) et partie inférieure (1b) de pas plus de 30kg étant portatives manuellement et indépendantes et séparables manuellement l'une de l'autre ; et
- un compartiment de rangement additionnel (5) contenant ou apte à contenir du matériel pour réaliser des prélèvements d'échantillon de fluide ou tissu corporel approprié et/ou du matériel et réactifs utiles pour réaliser les dits test d'analyse microbiologiques, ainsi qu'une poubelle (4) pour déchets biologiques, le dit compartiment de rangement additionnel (5) étant constitué par une zone ouverte en face avant de ladite partie supérieure , située dessous la dite enceinte , entre ladite partie isolée thermique (6) et la dite enceinte, le corps de la dite poubelle étant situé dessous le plan de travail de la dite enceinte.

4. Dispositif selon l'une des revendications 1 à 3 **caractérisé en ce que** les m trousses de tests d'analyse biologique de chaque dite catégorie de syndromes de dites maladies, sont regroupés dans une même enveloppe présentant une caractéristique distinctive visuelle différente pour respectivement les différentes dites catégories de syndromes de dites maladies, et les dites enveloppes sont disposés chacune dans un compartiment cloisonné différent de la dite armoire réfrigérée, les dits compartiments cloisonnés présentant des caractéristiques distinctives visuelles différentes entre elles, ledit programme d'ordinateur étant apte à indiquer à l'étape e2) au moins un dit compartiment cloisonné contenant respectivement le dit type d'enveloppe contenant la ou les trousse de tests d'analyses biologiques à réaliser,

5. Dispositif selon l'une des revendications 1 à 4 **caractérisé en ce que** les dites trousses comprennent des réactifs et supports solides de tests immuno-chromatographiques dont les résultats sont révélés sur un dit support solide présentant une caractéristique visuelle différente selon que le test est positif ou négatif et sont des tests réalisables en pas plus de 1 heure.

6. Dispositif selon l'une des revendications 1 à 5, **caractérisé en ce que** es types d'enveloppes comprennent des dites trousses de tests de k=6 catégories de syndromes de dites maladies comprenant un classement des syndromes par le type d'organe pouvant être la cible de ladite infection, comprenant :
- la catégorie des maladies affectant le poumon pour le syndrome « affections respiratoire », et
- la catégorie des infections affectant le larynx ou la gorge pour le syndrome « angine »,
- la catégorie des infections affectant le tube digestif pour le syndrome « gastro entérite »,
- la catégorie des infections affectant les organes sexuels et/ou transmises par contact au niveau des sécrétions des organes sexuel pour le syndrome « infections sexuellement transmissibles», et
- la catégorie des infections de maladies tropicales affectant le sang pour le syndrome « fièvre tropicale », et
- la catégorie des infections affectant le tractus urinaire pour le syndrome « infection urinaire », et
- la catégorie des infections affectant le foie pour le syndrome « ictère ».

7. Dispositif selon la revendication 6, **caractérisé en ce que** les types d'enveloppes comprennent des dites trousses de tests de k=6 catégories de syndromes de dites maladies comprenant un classement des syndromes par le type d'organe pouvant être la cible de ladite infection par lesdits microorganismes pathogènes suivants pour les dites catégories d'infections suivantes :
- pour la catégorie des maladies affectant le poumon, les dits microorganismes sont choisis parmi : *Legianella pneumophila* (maladie des légionnaires), *Streptococcus pneumoniae,* virus *Influenza*; et virus respiratoire syncitial, et
- pour la catégorie des infections affectant le larynx ou la gorge, les dits microorganismes sont choisis parmi : *Streptococcus pyogenes* et le virus Epstein Barr; et
- pour la catégorie des infections affectant le tube digestif, les dits microorganismes sont choisis parmi les Norovirus, Adeno-rotavirus, *Clostridium difficile, Salmonella enterica thypi,* et *Vibrio cholerae*; et
- pour la catégorie des infections sexuellement transmissibles affectant les organes sexuels et/ou transmises par contact au niveau des sécrétions des organes sexuels, les dits microorganismes sont choisis *Chlamydia trachomatis, Neisseria gonorrhae, Treponema pallidum*; le virus de l'herpès, et
- pour la catégorie des infections de maladies dites de fièvres tropicales affectant le sang, les dits microorganismes sont choisis parmi : *Plasmodium falciparum,* les bactéries *Borrelia* des fièvres récurrentes, de préférence *Barrelia crocidurae* et *Borrelai duttonii,* le virus de la Dengue et le virus Chikgunuya; *Leptosira interrogans et Salmonella enterica thypi, et*
- pour la catégorie des infections affectant le tractus urinaire, les dits microorganismes sont choisis parmi : *Escherichia coli* et des bactéries *Proteus* spp., *Citrobacter* spp., *Enterobacter* spp. et *Klebsiella* spp. Et
- la catégorie des infections affectant .le foi pour le syndrome « ictère » ; les dits microorganismes sont choisis parmi : virus de l'hépatite B, virus de l'hépatite C, virus Epstein Barr, *Plasmodium falciparum, Leptosira interrogans et Salmonella enterica thypi.*

8. Dispositif selon l'une des revendications 1 à 7, **caractérisée en ce que** les types d'enveloppes comprennent des dites trousses de tests de catégories de syndromes de pathologies non infectieuses pour la catégorie des maladies suivantes affectant le poumon:
- « embolie pulmonaire » pour laquelle le test d'analyse est un test de détection rapide par dosage de dimères de fibrines dans le sang, et
- « infarctus du myocarde » pour lequel le test d'analyse est un test de détection rapide par dosage de troponine.

9. Méthode de mise en oeuvre d'un dispositif portatif et/ou mobile comprenant une cabine laboratoire (1) selon l'une des revendications 1 à 8 comprenant les étapes suivantes dans lesquelles :
- e1) on enregistre à l'aide dudit programme d'ordinateur la sélection du ou des signes ou symptômes ressentis par le patient choisis parmi n signes ou symptômes susceptibles d'être par des patients atteints d'au moins une des m pathologies de chacune des dites k catégories de syndromes de dites maladies, n'étant un nombre entier de préférence au moins égal à k, et
- e2) le dit programme d'ordinateur détermine et indique le ou les types de dites enveloppes contenant la (ou les) trousse(s) de test d'analyse biologique à réaliser en fonction de la dite sélection de l'étape e1), et
- e3) le dit programme d'ordinateur détermine et indique le (ou les) mode(s) opératoire(s) pour effectuer le (ou les) prélèvement(s) d'échantillon(s) biologique(s) de fluide ou sécrétion corporelle dudit patient à analyser à l'aide des matériels et réactifs contenus dans la (ou les) dites(s) enveloppe(s) déterminée(s) à l'étape2) et/ou dans ledit mobilier de laboratoire, et
- e4) le dit programme d'ordinateur détermine et indique le (ou les) mode(s) opératoire(s) pour réaliser et interpréter les résultats des dits test d'analyses biologiques contenus dans la (ou les) dite(s) enveloppes déterminée(s) à l'étape2), et
- e5) on enregistre à l'aide du dit programme d'ordinateur les résultats positif, négatif ou ininterprétable de chacun des dits tests d'analyses biologiques réalisés contenus dans la (ou les) dites(s) enveloppe(s) déterminée(s) à l'étape e2), et
- e6) le dit programme d'ordinateur détermine et indique les actions à entreprendre et/ou conduite à respecter pour le patient et/ou les personnes de son entourage selon les indications fournies par ledit programme d'ordinateur en fonction de la combinaison de résultats positifs ou négatifs ou ininterprétables de tous les tests réalisés enregistrés à l'étape e5).

10. Méthode de mise en oeuvre d'un dispositif portatif et/ou mobile comprenant une cabine laboratoire (1) selon la revendication 9 **caractérisé en ce qu'**on réalise les étapes suivantes :
- à l'étape e2), on saisit physiquement dans la dite armoire le (ou les) type(s) de dites enveloppes contenant la ou les trousse de test d'analyse biologique à réaliser qui sont indiquées par ledit programme d'ordinateur, et
- à l'étape e3), on place dans la dite enceinte des matériels et réactifs contenus dans la (ou les) dite(s) enveloppe(s) et/ou dans ledit mobilier de laboratoire selon le (ou les) dit(s) mode(s) opératoire(s) indiqué par ledit programme d'ordinateur pour effectuer au moins un prélèvement d'échantillon biologique de fluide ou sécrétion corporelle dudit patient à analyser, et
- à l'étape e4), on réalise dans l'espace confiné au sein de la dite enceinte le ou les dits tests d'analyses biologiques contenus dans la (ou les) dite(s) enveloppe(s) selon les indications de mode(s) opératoire(s) fournies par ledit programme d'ordinateur, et on évalue les résultats positif, négatif ou ininterprétable de chacun des dits tests d'analyses biologiques réalisés selon les indications fournies par ledit programme d'ordinateur, et
- à l'étape e6) on effectue les actions à entreprendre et/ou conduite à respecter pour le patient et/ou les personnes de son entourage selon les indications fournies par ledit programme d'ordinateur.

11. Méthode de mise en oeuvre d'un dispositif portatif et/ou mobile comprenant une cabine laboratoire (1) selon les revendications 9 ou 10 comprenant les étapes successives suivantes dans lesquelles :
- e1) ledit programme d'ordinateur affiche sur l'écran de l'ordinateur, n signes ou symptômes pouvant être ressentis par des patients atteints d'au moins une des m pathologies de chacune des dites k catégories de syndromes de dites maladies, n'étant un nombre entier de préférence au moins égal à k, et on sélectionne par contact tactile et/ou un clavier de l'ordinateur les signes cliniques et/ou symptômes ressentis par le patient, et
- e2) le dit programme d'ordinateur affiche sur l'écran de l'ordinateur le ou les types de dites enveloppes contenant la (ou les) trousse(s) de test d'analyse biologique à réaliser, et
- e3) le dit programme d'ordinateur affiche sur l'écran de l'ordinateur le mode opératoire pour effectuer au moins un prélèvement d'échantillon biologique de fluide ou sécrétion corporelle dudit patient à analyser, et
- e4) le dit programme d'ordinateur affiche sur l'écran de l'ordinateur le mode opératoire pour réaliser et interpréter les résultats, et
- e5) le dit programme d'ordinateur affiche sur l'écran de l'ordinateur les instructions pour enregistrer les résultats positif, négatif ou ininterprétable de chacun des dits tests d'analyses biologiques réalisés, et
- e6) le dit programme d'ordinateur affiche sur l'écran de l'ordinateur les actions à entreprendre et/ou conduite à respecter pour le patient et/ou les personnes de son entourage en fonction de la combinaison de résultats positifs ou négatifs ou ininterprétables de tous les tests réalisés.

12. Méthode selon l'une des revendications 9 à 11, **caractérisé en ce qu'**à l'étape e1), les n signes clinique et/ou symptômes comprennent au moins les signes ou symptômes suivants ou combinaison suivantes:
- mal au ventre, nausées, vomissements et/ou diarrhées, pour le syndrome « gastro entérite »,
- brulure en urinant et/ou fièvre, pour le syndrome « infections urinaires »,
- écoulement ou lésion de l'organe génital, notamment écoulement de la verge, pour le syndrome « infections sexuellement transmissibles»,
- toux et/ou douleur dans le thorax et/ou fièvre pour le syndrome « affections respiratoire »,
- mal à la gorge et/ou fièvre pour le syndrome « angine »,
- fièvre seulement sans autre symptôme ci-dessus, et avec séjour récent en région tropicale pour le syndrome « fièvre tropicale », et
- coloration jaune de la peau pour le syndrome « ictère ».

13. Méthode selon l'une des revendications 9 à 12 **caractérisé en ce qu'**à l'étape e1), chaque dit syndrome correspond à un signe clinique ou symptôme différent ou une combinaison différente de dits signes cliniques ou symptômes.

14. Méthode selon l'une des revendications 9 à 13 **caractérisé en ce qu'**à l'étape e6), les dites actions à entreprendre sont choisies parmi au moins :
- évacuation du patient en urgence vers un établissement approprié pour recevoir un traitement thérapeutique et/ou pour faire effectuer des analyses biologiques complémentaires, tel qu'un hôpital, et
- isolement du patient du fait d'une infection contagieuse et/ou mesures d'hygiène pour le patient et/ou les autres personnes de son entourage, et
- communication des résultats des tests d'analyses biologiques à un établissement de traitement à distance pour fourniture d'ordonnance de traitement du patient pour administration sur place et/ou communication d'une proposition de traitement du patient à démarrer sur place.

15. Méthode selon l'une des revendications 9 à 14 **caractérisé en ce que** la dite cabine est installée sur un navire et à l'étape e6), les instructions d'évacuation, comprennent des instructions de déroutement du navire.

## Patentansprüche

1. Laborvorrichtung für die Durchführung eines Diagnoseverfahrens, umfassend die Durchführung einer Reihe von biologischen In-vitro-Analysetests zur Diagnose von einen dringenden medizinischen Eingriff erfordernden akuten Krankheiten, die wenigstens eine Vielzahl von akuten Infektionskrankheiten umfassen, umfassend:
- k Typen von Hüllen, die jeweils m Testkits für die biologische Analyse enthalten, wobei die m Testkits für biologische Analysen jeweils den Nachweis von je m Pathologien von k Kategorien von Syndromen akuter Krankheiten ermöglichen, die wenigstens Kategorien von Syndromen von Infektionen durch pathologische Mikroorganismen umfassen, wobei die Kategorien von Syndromen von Krankheiten jeweils einem anderen Syndrom, das ein Körperorgan oder -gewebe betrifft, zugeordnet sind, wobei k eine ganze Zahl größer als 2 ist, wobei vorzugsweise k wenigstens gleich 6 Kategorien ist, wobei m eine ganze Zahl vorzugsweise von 1 bis 5 ist, wobei m für jede Syndromkategorie einer Krankheit verschieden sein kann,
- eine Umschließung (2), die mit einer Arbeitsfläche (2d) ausgestattet ist, innerhalb welcher Umschließung die biologischen In-vitro-Analysetests durchgeführt werden können, und
- ein Aufbewahrungsmöbel (5, 1b), das wenigstens einen wärmeisolierten Schrank (6) umfasst, wobei der Schrank eine Vielzahl von Hüllen, die jeweils wenigstens ein Testkit für die biologische Analyse enthalten, enthält oder geeignet ist, diese zu enthalten, wobei das Aufbewahrungsmöbel (5, 1b) unter der Umschließung angeordnet ist,
wobei die Vorrichtung **dadurch gekennzeichnet ist, dass** sie in Form einer tragbaren und/oder mobilen Kabine vorliegt, die durch eine einzige Person von Hand transportiert werden kann, umfassend:
- eine Umschließung (2), die vorderseitig durch eine abnehmbare oder schwenkbare transparente Abdeckhaube (3) verschlossen ist, die mit wenigstens einer Zugangsöffnung (3a) für die beiden Arme einer Bedienungsperson (10) versehen ist, innerhalb welcher Umschließung eine Bedienungsperson die biologischen In-vitro-Analysetests auf der Arbeitsfläche in stehender Position, in einem mit der Abdeckhaube umschlossenen Raum durchführen kann, und
- einen Computer (7), vorzugsweise in Form eines Tablet-PCs, der innerhalb der Umschließung oberhalb der Arbeitsfläche angeordnet ist, der mit einem Programm, das geeignet ist, die Bedienungsperson bei der Durchführung der biologischen Analysetests zu unterstützen, oder mit einem computerlesbaren Speichermedium, auf dem ein Computerprogramm gespeichert ist, ausgestattet ist; wobei das Computerprogramm ausgelegt ist für die Ausführung einer Reihe von Schritten, welche die Auswahl und Realisierung sowie die Auslegung der Ergebnisse der biologischen Analysetests und die Bestimmung der zu unternehmenden Aktionen und/oder des für den Patienten und/oder das Personal in seiner Umgebung zu achtenden Verhaltens in Abhängigkeit von den Ergebnissen der Tests leiten, umfassend wenigstens die Abfolge der nachfolgenden Schritte:
- e1) die Speicherung der Auswahl von klinischen Anzeichen und/oder Symptomen, die der Patient empfindet, die aus in dem Computerprogramm gespeicherten n Anzeichen oder Symptomen ausgewählt sind, die von Patienten empfunden werden können, welche an wenigstens einer der m Pathologien einer jeden der k Kategorien von Syndromen von Krankheiten leiden, wobei n eine ganze Zahl vorzugsweise wenigstens gleich k ist, und
- e2) die Bestimmung und die Angabe des Typs oder der Typen von Hüllen, die das (oder die) Kit(s) für einen biologischen Analysetest enthalten, welcher in Abhängigkeit von der Auswahl des Schrittes e1) durchzuführen ist, und
- e3) die Bestimmung und die Angabe der Vorgehensweise oder Vorgehensweisen zum Durchführen wenigstens einer Entnahme einer zu analysierenden biologischen Probe von Körperflüssigkeit oder -sekret des Patienten mit Hilfe der Materialien und Reagenzien, welche in der (oder den) bei Schritt e2) bestimmten Hülle(n) und/oder in dem Labormöbel enthalten sind, und
- e4) die Bestimmung und die Angabe der Vorgehensweise oder Vorgehensweisen zum Durchführen und Auslegen der Ergebnisse der biologischen Analysetests, die in der (oder den) in Schritt e2) bestimmten Hülle(n) enthalten sind, und
- e5) die Speicherung des positiven, negativen oder nicht auslegbaren Ergebnisses eines jeden der durchgeführten biologischen Analysetests, die in der (oder den) bei Schritt e2) bestimmten Hülle(n) enthalten sind, und
- e6) die Bestimmung und die Angabe der zu unternehmenden Aktionen und/oder des für den Patienten und/oder die Personen in seiner Umgebung zu achtenden Verhaltens in Abhängigkeit von der Kombination aus positiven oder negativen oder nicht auslegbaren Ergebnissen von allen durchgeführten, bei Schritt e5) gespeicherten Tests.

2. Vorrichtung nach Anspruch 1, **dadurch gekennzeichnet, dass** die Speicherungen und die Angaben der Schritte e1) bis e6) über eine graphische Schnittstelle des Bildschirms des Computers, mit der die Bedienungsperson durch Tastkontakt und/oder über eine Tastatur interagieren kann, vorgenommen oder bereitgestellt werden.

3. Vorrichtung nach Anspruch 1 oder 2, **dadurch gekennzeichnet, dass** die Laborkabine umfasst:
- einen oberen Teil (1a), umfassend die Umschließung (2) mit einer Abdeckhaube (3), die mit zwei ersten kreisförmigen Öffnungen (3a) versehen ist, welche durch eine flexible Wand verschlossen sind, die zum Einführen von zwei Armen einer stehenden Bedienungsperson von außerhalb der Umschließung durchgreifbar ist, und eine Arbeitsfläche (2d), die Bereiche aufweist, die durch Umfangsränder begrenzt sind, welche Hohlaufnahmen (2-3) mit spezifischen Formen bilden, die dazu ausgelegt sind, in jedem Bereich jeweils ein Material oder ein Reagens für die Durchführung der Tests aufzunehmen und an Ort und Stelle zu halten, sowie eine zweite Öffnung (2-4) zum Abführen von Abfällen zu einem Abfalleimer, wobei der Abfalleimer (4) dazu eingerichtet oder geeignet ist, mit der Umschließung derart angeordnet zu werden, dass eine Bedienungsperson durch die zweite Öffnung innerhalb der Umschließung einen Test-Abfall, wie zum Beispiel gebrauchtes Material, vom Innenraum der Umschließung, den ihre Hände im Bereich der ersten Öffnungen der Umschließung durchgreifen, direkt in den Abfalleimer abführen kann, ohne den Abfall der Außenatmosphäre der Umschließung und des Abfalleimers auszusetzen, und
- einen unteren Teil (1b), der das Aufbewahrungsmöbel (1b) umfasst, welches den unabhängigen und abnehmbaren gekühlten Schrank (6) umfasst, und der untere Teil geeignet ist, den oberen Teil zu tragen,
- wobei der obere Teil (1a) und der untere Teil (1b) von nicht mehr als 30 kg von Hand tragbar sind sowie unabhängig und manuell voneinander trennbar sind, und
- ein zusätzliches Aufbewahrungsfach (5), das Material für die Durchführung von Entnahmen einer geeigneten Probe von Körperflüssigkeit oder -gewebe und/oder Material und Reagenzien für die Durchführung der mikrobiologischen Analysetests, sowie einen Abfalleimer (4) für biologische Abfälle enthält oder geeignet ist, diese zu enthalten, wobei das zusätzliche Aufbewahrungsfach (5) durch einen vorderseitig offenen Bereich des oberen Teils, der unterhalb der Umschließung zwischen dem isolierten Wärmeteil (6) und der Umschließung gelegen ist, gebildet ist, wobei das Gehäuse des Abfalleimers unterhalb der Arbeitsfläche der Umschließung gelegen ist.

4. Vorrichtung nach einem der Ansprüche 1 bis 3, **dadurch gekennzeichnet, dass** die m Kits für biologische Analysetests einer jeden Kategorie von Syndromen von Krankheiten in einer gleichen Hülle zusammengefasst sind, die für jeweils die verschiedenen Kategorien von Syndromen von Krankheiten ein anderes visuelles Unterscheidungsmerkmal aufweist, und die Hüllen jeweils in einem von dem gekühlten Schrank verschiedenen, abgeteilten Fach angeordnet sind, wobei die abgeteilten Fächer untereinander verschiedene visuelle Unterscheidungsmerkmale aufweisen, wobei das Computerprogramm geeignet ist, bei Schritt e2) wenigstens ein abgeteiltes Fach anzuzeigen, das jeweils den Typ Hülle enthält, die das oder die Kits für durchzuführende biologische Analysetests enthält.

5. Vorrichtung nach einem der Ansprüche 1 bis 4, **dadurch gekennzeichnet, dass** die Kits Reagenzien und feste Träger für immunochromatographische Tests umfassen, deren Ergebnisse auf einem festen Träger mit unterschiedlichem visuellem Merkmal in Abhängigkeit davon, ob der Test positiv oder negativ ist, angezeigt werden, und Tests sind, die in nicht mehr als 1 Stunde durchführbar sind.

6. Vorrichtung nach einem der Ansprüche 1 bis 5, **dadurch gekennzeichnet, dass** die Typen von Hüllen Testkits von k = 6 Kategorien von Krankheitssyndromen umfassen, die eine Klassifizierung der Syndrome nach der Art des Organs, das das Ziel der Infektion sein kann, umfassen, umfassend:
- die Kategorie von Krankheiten, die die Lunge betreffen, für das Syndrom "Atemwegserkrankungen", und
- die Kategorie von Infektionen, die den Kehlkopf oder den Hals betreffen, für das Syndrom "Angina",
- die Kategorie von Infektionen, die den Verdauungstrakt betreffen, für das Syndrom "Gastroenteritis",
- die Kategorie von Infektionen, die die Geschlechtsorgane betreffen und/oder durch Kontakt im Bereich der Sekrete der Geschlechtsorgane übertragen werden, für das Syndrom "sexuell übertragbare Infektionen", und
- die Kategorie von Infektionen von tropischen Erkrankungen, die das Blut betreffen, für das Syndrom "tropisches Fieber", und
- die Kategorie von Infektionen, die die Harnwege betreffen, für das Syndrom "Harnwegsinfektion", und
- die Kategorie von Infektionen, die die Leber betreffen, für das Syndrom "Gelbsucht".

7. Vorrichtung nach Anspruch 6, **dadurch gekennzeichnet, dass** die Typen von Hüllen Testkits von k = 6 Kategorien von Krankheitsyndromen, mit einer Klassifizierung der Syndrome nach dem Typ des Organs, das das Ziel der Infektion durch die folgenden pathogenen Mikroorganismen sein kann, für die folgenden Infektionskategorien umfassen:
- für die Kategorie von Krankheiten, die die Lunge betreffen, sind die Mikroorganismen ausgewählt aus: *Legionella pneumophila* (Legionärskrankheit), *Streptococcus pneumoniae,* Influenzavirus; und respiratorisches Synzitial-Virus und
- für die Kategorie von Infektionen, die den Kehlkopf oder den Hals betreffen, sind die Mikroorganismen ausgewählt aus: *Streptococcus pyogenes* und dem Epstein-Barr-Virus, und
- für die Kategorie von Infektionen, die den Verdauungstrakt betreffen, sind die Mikroorganismen ausgewählt aus Norovirus, Adeno-Rotavirus, *Clostridium difficile, Salmonella enterica thypi* und *Vibrio cholerae,* und
- für die Kategorie von sexuell übertragbaren Infektionen, die die Geschlechtsorgane betreffen und/oder durch Kontakt im Bereich der Sekrete der Geschlechtsorgane übertragen werden, sind die Mikroorganismen ausgewählt aus *Chlamydia trachomatis, Neisseria gonorrhae, Treponema pallidum,* dem Herpesvirus, und
- für die Kategorie von Infektionen sogenannter tropischer Fieberkrankheiten, die das Blut betreffen, sind die Mikroorganismen ausgewählt aus: *Plasmodium falciparum,* Borrelienbakterien von Rückfallfieber, vorzugsweise *Borrelia crocidurae* und *Borrelai duttonii,* dem Dengue-Virus und dem Chikgunuya-Virus; *Leptosira interrogans* und *Salmonella enterica thypi,* und
- für die Kategorie von Infektionen, die die Harnwege betreffen, sind die Mikroorganismen ausgewählt aus: *Escherichia coli und* Bakterien *Proteus* spp., *Citrobacter* spp., *Enterobacter* spp. und *Klebsiella* spp., und
- die Kategorie von die Leber betreffenden Infektionen, für das "Gelbsucht"-Syndrom, sind die Mikroorganismen ausgewählt aus: dem Hepatitis-B-Virus, dem Hepatitis-C-Virus, dem Epstein-Barr-Virus, *Plasmodium falciparum, Leptosira interrogans* und *Salmonella enterica thypi.*

8. Vorrichtung nach einem der Ansprüche 1 bis 7, **dadurch gekennzeichnet, dass** die Typen von Hüllen Testkits von Kategorien von Syndromen nicht-infektiöser Pathologien für die Kategorie der folgenden, die Lunge betreffenden Krankheiten umfassen:
- "Lungenembolie", für die der Analysetest ein Schnellerkennungstest durch Bestimmung von Fibrindimeren im Blut ist, und
- "Herzinfarkt", für den der Analysetest ein Schnellerkennungstest durch Troponin-Bestimmung ist.

9. Verfahren zum Verwenden einer tragbaren und/oder mobilen Vorrichtung, die eine Laborkabine (1) nach einem der Ansprüche 1 bis 8 umfasst, umfassend die folgenden Schritte, bei denen:
- e1) mit Hilfe des Computerprogramms die Auswahl des oder der Anzeichen und/oder Symptome, die der Patient empfindet, ausgewählt aus n Anzeichen oder Symptomen, die von Patienten empfunden werden können, welche an wenigstens einer der m Pathologien einer jeden der k Kategorien von Syndromen von Krankheiten leiden, gespeichert wird, wobei n eine ganze Zahl vorzugsweise wenigstens gleich k ist, und
- e2) das Computerprogramm den Typ oder die Typen von Hüllen bestimmt und angibt, die das (oder die) Kit(s) für einen biologischen Analysetest enthalten, welcher in Abhängigkeit von der Auswahl des Schrittes e1) durchzuführen ist, und
- e3) das Computerprogramm die Vorgehensweise (oder Vorgehensweisen) zum Durchführen der Entnahme (oder Entnahmen) einer (von) zu analysierenden biologischen Probe(n) von Körperflüssigkeit oder -sekret des Patienten mit Hilfe der Materialien und Reagenzien, welche in der (oder den) in Schritt e2) bestimmten Hülle(n) und/oder in dem Labormöbel enthalten ist (sind), bestimmt und angibt, und
- e4) das Computerprogramm die Vorgehensweise (oder Vorgehensweisen) zum Durchführen und Auslegen der Ergebnisse der biologischen Analysetests, die in der (oder den) in Schritt e2) bestimmten Hülle(n) enthalten sind, bestimmt und angibt, und
- e5) mit Hilfe des Computerprogramms die positiven, negativen oder nicht auslegbaren Ergebnisse eines jeden der durchgeführten biologischen Analysetests, die in der (oder den) bei Schritt e2) bestimmten Hülle(n) enthalten sind, gespeichert werden, und
- e6) das Computerprogramm die zu unternehmenden Aktionen und/oder das für den Patienten und/oder die Personen in seiner Umgebung zu achtende Verhalten entsprechend den Angaben bestimmt und angibt, die durch das Computerprogramm in Abhängigkeit von der Kombination aus positiven oder negativen oder nicht auslegbaren Ergebnissen von allen durchgeführten, bei Schritt e5) gespeicherten Tests bereitgestellt werden.

10. Verfahren zum Verwenden einer tragbaren und/oder mobilen Vorrichtung mit einer Laborkabine (1), nach Anspruch 9, **dadurch gekennzeichnet, dass** die folgenden Schritte durchgeführt werden:
- bei Schritt e2) werden in dem Schrank der (oder die) Typ(en) von Hüllen, welche das oder die Kits für einen durchzuführenden biologischen Analysetest enthalten, die durch das Computerprogramm angezeigt werden, physisch erfasst, und
- bei Schritt e3) werden in der Umschließung Materialien und Reagenzien, die in der (den) Hülle(n) und/oder in dem Labormöbel enthalten sind, gemäß der durch das Computerprogramm angegebenen Vorgehensweise(n) platziert, um wenigstens eine Entnahme einer zu analysierenden biologischen Probe von Körperflüssigkeit oder - sekret des Patienten zu vollziehen, und
- bei Schritt e4) werden in dem umschlossenen Raum innerhalb der Umschließung der oder die Tests für biologische Analysen, welche in der (oder den) Hülle(n) enthalten sind, entsprechend den Angaben zu(r) Vorgehensweise(n), die durch das Computerprogramm bereitgestellt werden, durchgeführt und werden die positiven, negativen oder nicht auslegbaren Ergebnisse eines jeden der durchgeführten biologischen Analysetests entsprechend den durch das Computerprogramm bereitgestellten Angaben ausgewertet, und
- bei Schritt e6) werden die zu unternehmenden Aktionen und/oder das für den Patienten und/oder die Personen in seiner Umgebung zu achtende Verhalten entsprechend den durch das Computerprogramm bereitgestellten Angaben durchgeführt bzw. umgesetzt.

11. Verfahren zum Verwenden einer tragbaren und/oder mobilen Vorrichtung mit einer Laborkabine (1), nach den Ansprüchen 9 oder 10, umfassend die folgenden aufeinanderfolgenden Schritte, bei denen:
- e1) das Computerprogramm auf dem Bildschirm des Computers n Anzeichen oder Symptome, die von Patienten empfunden werden können, welche an wenigstens einer der m Pathologien einer jeden der k Kategorien von Syndromen von Krankheiten leiden, anzeigt, wobei n eine ganze Zahl vorzugsweise wenigstens gleich k ist, und durch Tastkontakt und/oder eine Tastatur des Computers die klinischen Anzeichen und/oder Symptome, welche durch den Patienten empfunden werden, ausgewählt werden, und
- e2) das Computerprogramm auf dem Bildschirm des Computers den Typ oder die Typen von Hüllen anzeigt, die das (oder die) Kit(s) für einen durchzuführenden biologischen Analysetest enthalten, und
- e3) das Computerprogramm auf dem Bildschirm des Computers die Vorgehensweise zum Durchführen wenigstens einer Entnahme einer zu analysierenden biologischen Probe von Körperflüssigkeit oder -sekret des Patienten anzeigt, und
- e4) das Computerprogramm auf dem Bildschirm des Computers die Vorgehensweise für die Durchführung und Auslegung der Ergebnisse anzeigt, und
- e5) das Computerprogramm auf dem Bildschirm des Computers die Anweisungen zum Speichern der positiven, negativen oder nicht auslegbaren Ergebnisse eines jeden der durchgeführten biologischen Analysetests anzeigt, und
- e6) das Computerprogramm auf dem Bildschirm des Computers die zu unternehmenden Aktionen und/oder das für den Patienten und/oder die Personen in seiner Umgebung zu achtende Verhalten in Abhängigkeit von der Kombination aus positiven oder negativen oder nicht auslegbaren Ergebnissen von allen durchgeführten Tests anzeigt.

12. Verfahren nach einem der Ansprüche 9 bis 11, **dadurch gekennzeichnet, dass** bei Schritt e1) die n klinischen Anzeichen und/oder Symptome wenigstens die folgenden Anzeichen oder Symptome oder folgenden Kombinationen umfassen:
- Bauchschmerzen, Übelkeit, Erbrechen und/oder Durchfall, für das Syndrom "Gastroenteritis",
- Brennen beim Wasserlassen und/oder Fieber, für das Syndrom "Harnwegsinfektionen",
- Ausfluss oder Verletzung des Geschlechtsorgans, insbesondere Ausfluss aus dem Penis, für das Syndrom "sexuell übertragbare Infektionen",
- Husten und/oder Schmerzen im Brustkorb und/oder Fieber, für das Syndrom "Atemwegserkrankungen",
- Halsschmerzen und/oder Fieber, für das Syndrom «Angina»,
- Fieber, nur ohne anderes obiges Symptom, und mit kürzlichem Aufenthalt in tropischer Region, für das Syndrom "tropisches Fieber", und
- Gelbfärbung der Haut, für das Syndrom «Gelbsucht».

13. Verfahren nach einem der Ansprüche 9 bis 12, **dadurch gekennzeichnet, dass** bei Schritt e1) jedes Syndrom einem anderen klinischen Anzeichen und/oder Symptom oder einer anderen Kombination von klinischen Anzeichen oder Symptomen entspricht.

14. Verfahren nach einem der Ansprüche 9 bis 13, **dadurch gekennzeichnet, dass** bei Schritt e6) die zu unternehmenden Aktionen ausgewählt sind aus wenigstens:
- dringender Abtransport des Patienten zu einer geeigneten Einrichtung, um eine therapeutische Behandlung zu erhalten und/oder um ergänzende biologische Analysen durchführen zu lassen, wie einem Krankenhaus, und
- Isolieren des Patienten aufgrund einer ansteckenden Infektion und/oder Hygienemaßnahmen für den Patienten und/oder die anderen Personen in seiner Umgebung, und
- Mitteilen der Ergebnisse der biologischen Analysetests an eine entfernte Behandlungseinrichtung zur Bereitstellung eines Rezepts für die Behandlung des Patienten für eine Vor-Ort-Verabreichung und/oder Mitteilung eines Vorschlags zur Behandlung des Patienten, die vor Ort zu beginnen ist.

15. Verfahren nach einem der Ansprüche 9 bis 14, **dadurch gekennzeichnet, dass** die Kabine auf einem Schiff installiert ist und bei Schritt e6) die Evakuierungsanweisungen Anweisungen zum Umleiten des Schiffes umfassen.

## Claims

1. A laboratory device useful for implementing a diagnostic method comprising performing a series of in vitro bioanalytical tests for the diagnosis of acute diseases requiring urgent medical intervention comprising at least a plurality of acute infectious diseases comprising:
- k types of envelopes each containing m bioanalytical test kits, said m bioanalytical test kits each allowing the detection of respectively m pathologies of k categories of syndromes of said acute diseases comprising at least categories of syndromes of infections by pathological microorganisms, said categories of syndromes of said diseases each being associated with a different syndrome affecting a body organ or tissue, k being an integer greater than 2, preferably k being at least equal to 6 categories, m being an integer preferably from 1 to 5, m which may be different for each said category of syndrome of said disease,
- an enclosure (2) equipped with a work surface (2d), within which enclosure said in vitro bioanalytical tests can be performed, and
- a storage furniture unit (5, 1b) comprising at least one thermally insulated cabinet (6), said cabinet containing or capable of containing a plurality of said envelopes each containing at least one said bioanalytical test kit, said storage furniture unit (5, 1b) being disposed under said enclosure,
The device being **characterized in that** it is in the form of a portable and/or mobile booth, manually transportable by a single person, comprising:
- an enclosure (2) closed on the front side by a removable or pivoting transparent hood (3), provided with at least one access opening (3a) for the two arms of an operator (10), within which enclosure an operator can perform said in vitro bioanalytical tests on said work surface in a standing position, in a space confined with said hood, and
- a computer (7), preferably in the form of a tablet, disposed within said enclosure above said work surface, equipped with a program capable of assisting the operator in performing said bioanalytical tests or with a recording medium readable by said computer on which a said computer program is recorded; said computer program being configured to execute a series of steps guiding the choice and the production as well as the interpretation of the results of said bioanalytical tests and the determination of the actions to be taken and/or the management guidelines for the patient and/or persons close to the patient as a function of the test results, comprising at least the succession of said following steps:
- s1) recording the selection of clinical signs and/or symptoms experienced by the patient selected from n signs or symptoms recorded in said computer program, which may be experienced by patients suffering from at least one of the m pathologies of each of said k categories of syndromes of said diseases, n being an integer preferably at least equal to k, and
- s2) determining and indicating the type(s) of said envelopes containing the bioanalytical test kit(s) to be used as a function of said selection from step s1), and
- s3) determining and indicating the procedure(s) for collecting at least one biological sample of body fluid or secretion from said patient to be analysed, using the equipment and reagents contained in said envelope(s) determined in step 2) and/or in said laboratory furniture unit, and
- s4) determining and indicating the procedure(s) for producing and interpreting the results of said bioanalytical tests contained in said envelope (s) determined in step s2), and
- s5) recording the positive, negative or uninterpretable result of each of said bioanalytical tests performed contained in said envelope(s) determined in step s2), and
- s6) determining and indicating the actions to be taken and/or the management guidelines for the patient and/or persons close to the patient as a function of the combination of positive or negative or uninterpretable results of all the tests performed recorded in step s5).

2. The device according to claim 1, **characterized in that** said recordings and said indications of steps s1) to s6) are carried out or provided via a graphical interface of the screen of said computer with which said operator can interact via touch and/or a keyboard.

3. The device according to claim 1 or 2, **characterized in that** said laboratory booth comprises:
- an upper part (1a) comprising said enclosure (2) with a said hood (3) provided with two first circular openings (3a) closed by a flexible wall that can be crossed from outside the enclosure to introduce the two arms of an operator in a standing position, and a said work surface (2d) comprising zones delimited by peripheral edges forming hollow housings (2-3) of specific shapes and suited to receive and hold in place in each said zone respectively an equipment item or a reagent useful for performing said tests, and a second opening (2-4) for discarding waste into a bin, said bin (4) being arranged or capable of being arranged with said enclosure so that an operator can discard directly into said bin, through said second opening within said enclosure, test waste such as used equipment, from inside the enclosure into which the operator's hands pass through said first openings of the enclosure, without exposing said waste to the atmosphere outside said enclosure and said bin, and
- a lower part (1b) comprising said storage furniture unit (1b) comprising said independent and removable refrigerated cabinet (6), and said lower part is capable of supporting said upper part,
- said upper part (1a) and lower part (1b) of not more than 30kg being manually portable and independent and manually separable from each other; and
- an additional storage compartment (5) containing or capable of containing equipment for collecting samples of suitable body fluid or tissue and/or equipment and reagents useful for performing said microbioanalytical tests, as well as a bin (4) for biological waste, said additional storage compartment (5) consisting of an area open on the front side of said upper part, located below said enclosure, between said thermally insulated part (6) and said enclosure, the main part of said bin being located below the work surface of said enclosure.

4. The device according to one of claims 1 to 3, **characterized in that** the m bioanalytical test kits of each said category of syndromes of said diseases are grouped together in the same envelope having a different distinctive visual feature for respectively the different said categories of syndromes of said diseases, and said envelopes are each disposed in a partitioned compartment different from said refrigerated cabinet, said partitioned compartments having different distinctive visual features between them, said computer program being capable of indicating in step s2) at least one said partitioned compartment containing respectively said type of envelope containing the bioanalytical test kit(s) to be used.

5. The device according to one of claims 1 to 4, **characterized in that** said kits comprise reagents and solid supports for immunochromatographic tests whose results are revealed on a said solid support having a different visual feature depending on whether the test is positive or negative and are tests that can be performed in no more than 1 hour.

6. The device according to one of claims 1 to 5, **characterized in that** the types of envelopes comprise said test kits of k=6 categories of syndromes of said diseases comprising a classification of syndromes by the type of organ that may be the target of said infection, comprising:
- the category of diseases affecting the lung for the "respiratory conditions" syndrome, and
- the category of infections affecting the larynx or throat for the "pharyngitis" syndrome,
- the category of infections affecting the digestive tract for the "gastroenteritis" syndrome,
- the category of infections affecting the sexual organs and/or transmitted by contact with the secretions of the sexual organs for the "sexually transmitted infections" syndrome, and
- the category of tropical disease infections affecting the blood for the "tropical fever" syndrome, and
- the category of infections affecting the urinary tract for the "urinary infection" syndrome, and
- the category of infections affecting the liver for the "jaundice" syndrome.

7. The device according to claim 6, **characterized in that** the types of envelopes comprise said test kits of k=6 categories of syndromes of said diseases comprising a classification of syndromes by the type of organ that may be the target of said infection by said following pathogenic microorganisms for said following categories of infections:
- for the category of diseases affecting the lung, said microorganisms are selected from: *Legionella pneumophila* (legionnaires' disease), *Streptococcus pneumoniae, Influenza* virus; and respiratory syncytial virus, and
- for the category of infections affecting the larynx or throat, said microorganisms are selected from: *Streptococcus pyogenes* and the Epstein-Barr virus; and
- for the category of infections affecting the digestive tract, said microorganisms are selected from Norovirus, Adeno-rotavirus, *Clostridium difficile, Salmonella enterica typhi,* and *Vibrio cholerae;* and
- for the category of sexually transmitted infections affecting the sexual organs and/or transmitted by contact with the secretions of the sexual organs, said microorganisms are selected *Chlamydia trachomatis, Neisseria gonorrhoeae, Treponema pallidum*; the herpes virus, and
- for the category of infections of diseases referred to as tropical fevers affecting the blood, said microorganisms are selected from: *Plasmodium falciparum,* relapsing fever *Borrelia* bacteria, preferably *Borrelia crocidurae* and *Borrelia duttoni,* Dengue virus and Chikungunya virus; *Leptospira interrogans* and *Salmonella enterica typhi,* and
- for the category of infections affecting the urinary tract, said microorganisms are selected from: *Escherichia coli* and bacteria *Proteus* spp., *Citrobacter* spp., *Enterobacter* spp. and *Klebsiella* spp.
- the category of infections affecting the liver for the "jaundice" syndrome; said microorganisms are selected from: hepatitis B virus, hepatitis C virus, Epstein-Barr virus, *Plasmodium falciparum, Leptospira interrogans* and *Salmonella enterica typhi.*

8. A device according to one of claims 1 to 7, **characterized in that** the types of envelopes comprise said test kits for categories of syndromes of non-infectious diseases for the category of the following diseases affecting the lung:
- "pulmonary embolism" for which the analytical test is a rapid detection test by blood fibrin dimer assay, and
- "myocardial infarction" for which the analytical test is a rapid detection test by troponin assay.

9. A method for implementing a portable and/or mobile device comprising a laboratory booth (1) according to one of claims 1 to 8 comprising the following steps in which:
- s1) the selection of the sign(s) or symptom(s) experienced by the patient selected from n signs or symptoms likely to be experienced by patients suffering from at least one of the m pathologies of each of said k categories of syndromes of said diseases, n being an integer preferably at least equal to k, is recorded using said computer program, and
- s2) said computer program determines and indicates the type(s) of said envelopes containing the bioanalytical test kit(s) to be used as a function of said selection from step s1), and
- s3) said computer program determines and indicates the procedure(s) for collecting the biological sample(s) of body fluid or secretion from said patient to be analysed using the equipment and reagents contained in said envelope(s) determined in step s2) and/or in said laboratory furniture unit, and
- s4) said computer program determines and indicates the procedure(s) for producing and interpreting the results of said bioanalytical tests contained in said envelope(s) determined in step s2), and
- s5) the positive, negative or uninterpretable results of each of said bioanalytical tests contained in the envelope(s) determined in step s2) are recorded using said computer program, and
- s6) said computer program determines and indicates the actions to be taken and/or the management guidelines for the patient and/or persons close to the patient according to the indications provided by said computer program as a function of the combination of positive or negative or uninterpretable results of all the tests performed recorded in step s5).

10. The method for implementing a portable and/or mobile device comprising a laboratory booth (1) according to claim 9, **characterized in that** the following steps are carried out:
- in step s2), the type(s) of envelopes containing the bioanalytical test kit(s) to be used which are indicated by said computer program are physically taken in hand inside said cabinet, and
- in step s3), the equipment and reagents contained in said envelope(s) and/or in said laboratory furniture unit are placed in said enclosure according to said procedure(s) indicated by said computer program for collecting at least one biological sample of body fluid or secretion from said patient to be analysed, and
- in step s4), said bioanalytical test(s) contained in said envelope(s) are performed in the confined space within said enclosure according to the procedural instructions provided by said computer program, and the positive, negative or uninterpretable results of each of said bioanalytical tests performed according to the instructions provided by said computer program are evaluated; and
- in step s6) the actions to be taken and/or the management guidelines for the patient and/or people close to the patient are carried out according to the instructions provided by said computer program.

11. The method for implementing a portable and/or mobile device comprising a laboratory booth (1) according to claims 9 or 10, comprising the following successive steps in which:
- s1) said computer program displays on the computer screen, n signs or symptoms that may be experienced by patients suffering from at least one of the m pathologies of each of said k categories of syndromes of said diseases, n being an integer preferably at least equal to k, and the clinical signs and/or symptoms experienced by the patient are selected via touch and/or a computer keyboard, and
- s2) said computer program displays on the computer screen the type(s) of said envelopes containing the bioanalytical test kit(s) to be used, and
- s3) said computer program displays on the computer screen the procedure for performing at least one collection of a biological sample of body fluid or secretion from said patient to be analysed, and
- s4) said computer program displays on the computer screen the procedure for producing and interpreting the results, and
- s5) said computer program displays on the computer screen the instructions for recording the positive, negative or uninterpretable results of each of said bioanalytical tests performed, and
- s6) said computer program displays on the computer screen the actions to be taken and/or the management guidelines for the patient and/or persons close to the patient as a function of the combination of positive or negative or uninterpretable results of all the tests performed.

12. The method according to one of claims 9 to 11, **characterized in that** in step s1), the n clinical signs and/or symptoms comprise at least the following signs or symptoms or combination thereof:
- stomach pain, nausea, vomiting and/or diarrhoea, for the "gastroenteritis" syndrome,
- burning during urination and/or fever, for the "urinary infections" syndrome,
- genital discharge or lesion, notably penile discharge, for the "sexually transmitted infections" syndrome,
- cough and/or chest pain and/or fever for the "respiratory conditions" syndrome,
- sore throat and/or fever for the "pharyngitis" syndrome,
- fever alone with no other symptom above, and with a recent stay in a tropical region for the "tropical fever" syndrome, and
- yellow colouring of the skin for the "jaundice" syndrome.

13. The method according to one of claims 9 to 12, **characterized in that** in step s1), each said syndrome corresponds to a different clinical sign or symptom or a different combination of said clinical signs or symptoms.

14. The method according to one of claims 9 to 13, **characterized in that** in step s6), said actions to be taken are selected from at least:
- emergency evacuation of the patient to an appropriate facility to receive therapeutic treatment and/or to carry out further bioanalyses, such as a hospital, and
- isolation of the patient due to a contagious infection and/or hygiene measures for the patient and/or other persons close to the patient, and
- communication of the results of the bioanalytical tests to a remote treatment facility for provision of the treatment prescription for the patient for on-site administration and/or communication of a treatment proposal for the patient to be started on-site.

15. The method according to one of claims 9 to 14, **characterized in that** said booth is installed on a ship and in step s6), the evacuation instructions include instructions for rerouting the ship.
